# EUROPEAN PATENT SPECIFICATION

(11) **EP 0 632 134 B1**
(45) Date of publication and mention of the grant of the patent: **05.09.2001**
(21) Application number: 94109468.2
(22) Date of filing: 20.06.1994
(51) Int. Cl.: C12Q 1/68

(54) **Reagents and methods for coupled high temperature reverse transcription and polymerase chain reaction**
Reagenz und Verfahren für reverse Transkription bei hoher Temperatur, verbunden mit einer Polymerase-Kettenreaktion
Réactif et procédé pour la transcription inverse à haute température suivi par la réaction en chaîne de la polymérase

(30) Priority: 01.07.1993 US 86483
(43) Date of publication of application: 04.01.1995
(73) Proprietor: F. HOFFMANN-LA ROCHE AG, 4002 Basel (CH)
(72) Inventor: Gelfand,David H, Oakland CA 94611 (US); Myers,Thomas W, Alameda CA 94501 (US); Sigua,Christopher L, Antioch CA 94509 (US)
(74) Representative: Knauer, Martin, Dr.

(56) References cited:
- EP-A- 0 529 493
- WO-A-91/09944
- WO-A-92/01814
- BIOCHEMISTRY, vol. 5, no. 2, 2 February 1966 BALTIMORE US, pages 467-477, N.E. GOOD ET AL.
- PROCEEDINGS OF THE NATIONAL ACADEMY OF SCIENCES OF USA, vol. 86, June 1989 WASHINGTON US, pages 4076-4080, S. TABOR ET AL.
- ABSTRACTS OF THE GENERAL MEETING OF THE AMERICAN SOCIETY FOR MICROBIOLOGY, vol. 94, no. 0, May 1994 WASHINGTON US, page 551 C.L. SIGUA ET AL.

### SEQUENCE LISTING

(1) GENERAL INFORMATION:
   (i) APPLICANT:
      (A) NAME: F.Hoffmann-La Roche AG
      (B) STREET: Grenzacherstrasse 124
      (C) CITY: Basel
      (D) STATE: BS
      (E) COUNTRY: Switzerland
      (F) POSTAL CODE (ZIP): CH-4002
      (G) TELEPHONE: (0)61 688 24 03
      (H) TELEFAX: (0)61 688 13 95
      (I) TELEX: 962292/965542 hlr ch
   (ii) TITLE OF INVENTION: Reagents and methods for coupled high temperature reverse transcription and polymerase chain reactions
   (iii) NUMBER OF SEQUENCES: 19
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.25 (EPO)
   (vi) PRIOR APPLICATION DATA:
      (A) APPLICATION NUMBER: US 08/086,483
      (B) FILING DATE: 01-JUL-1993
(2) INFORMATION FOR SEQ ID NO: 1:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: genomic DNA
   (xi) SEQUENCE DESCRIPTION: SEQ ID NO: 1:
   GGCATATGGC TAGACTATTT CTTTTTG 27
(2) INFORMATION FOR SEQ ID NO: 2:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: genomic DNA
   (xi) SEQUENCE DESCRIPTION: SEQ ID NO: 2:
   AGGTTCCGAT GAAGTCTGTA GGTGATGTCT G 31
(2) INFORMATION FOR SEQ ID NO: 3:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: genomic DNA
   (xi) SEQUENCE DESCRIPTION: SEQ ID NO: 3:
   CTACAGACTT CATCGGAACC TCCTTAAGCG 30
(2) INFORMATION FOR SEQ ID NO: 4:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: genomic DNA
   (xi) SEQUENCE DESCRIPTION: SEQ ID NO: 4:
   CCAACCCGCC TCGGCCACGA AGG 23
(2) INFORMATION FOR SEQ ID NO: 5:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: genomic DNA
   (xi) SEQUENCE DESCRIPTION: SEQ ID NO: 5:
   AGTTGGAGGA CATCAAGCAG CCATGCAAAT                                    30
(2) INFORMATION FOR SEQ ID NO: 6:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: genomic DNA
   (xi) SEQUENCE DESCRIPTION: SEQ ID NO: 6:
   TGCTATGTCA GTTCCCCTTG GTTCTCT                                       27
(2) INFORMATION FOR SEQ ID NO: 7:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: genomic DNA
   (xi) SEQUENCE DESCRIPTION: SEQ ID NO: 7:
   ATAATCCACC TATCCCAGTA GGAGAAAT                                      28
(2) INFORMATION FOR SEQ ID NO: 8:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: genomic DNA
   (xi) SEQUENCE DESCRIPTION: SEQ ID NO: 8:
   TTTGGTCCTT GTCTTATGTC CAGAATGC                                      28
(2) INFORMATION FOR SEQ ID NO: 9:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: genomic DNA
   (xi) SEQUENCE DESCRIPTION: SEQ ID NO: 9:
   TGGAGAACAC CACTTGTTGC TCCA                                          24
(2) INFORMATION FOR SEQ ID NO: 10:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: genomic DNA
   (xi) SEQUENCE DESCRIPTION: SEQ ID NO: 10:
   GTCTCTGAAT CAGAAATCCT TCTATC                                        26
(2) INFORMATION FOR SEQ ID NO: 11:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: genomic DNA
   (xi) SEQUENCE DESCRIPTION: SEQ ID NO: 11:
   CATGTCAAAT TTCACTGCTT CATCC                                         25
(2) INFORMATION FOR SEQ ID NO: 12:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: genomic DNA
   (xi) SEQUENCE DESCRIPTION: SEQ ID NO: 12:
   GCTTGCAAGC TTTATTTAGT TATGACTGAT AACACTC                            37
(2) INFORMATION FOR SEQ ID NO: 13:
   (i) SEQUENCE CHARACTERISTICS:
      (A) LENGTH: 44 base pairs
      (B) TYPE: nucleic acid
      (C) STRANDEDNESS: single
      (D) TOPOLOGY: linear
   (ii) MOLECULE TYPE: genomic DNA
   (xi) SEQUENCE DESCRIPTION: SEQ ID NO: 13:
   CAGGTCTCCC AAGTCTGGCG CCCTGCAAAT GAGACACTTT CTCG 44
(2) INFORMATION FOR SEQ ID NO: 14:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: genomic DNA
   (xi) SEQUENCE DESCRIPTION: SEQ ID NO: 14:
   GATCTCCGGA CTCTAGA 17
(2) INFORMATION FOR SEQ ID NO: 15:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: genomic DNA
   (xi) SEQUENCE DESCRIPTION: SEQ ID NO: 15:
   AATTTCTAGA GTCCGGA 17
(2) INFORMATION FOR SEQ ID NO: 16:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: genomic DNA
   (xi) SEQUENCE DESCRIPTION: SEQ ID NO: 16:
   CTCGCAAGCA CCCTATCAGG CAGT 24
(2) INFORMATION FOR SEQ ID NO: 17:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: genomic DNA
   (xi) SEQUENCE DESCRIPTION: SEQ ID NO: 17:
   GCAGAAAGCG TCTAGCCATG GCGT                                        24
(2) INFORMATION FOR SEQ ID NO: 18:
   (i) SEQUENCE CHARACTERISTICS:
      (A) LENGTH: 79 base pairs
      (B) TYPE: nucleic acid
      (C) STRANDEDNESS: single
      (D) TOPOLOGY: linear
   (ii) MOLECULE TYPE: genomic DNA
   (xi) SEQUENCE DESCRIPTION: SEQ ID NO: 18:
   CGCGTCGACA GTTGGAGGAC ATCAAGCAGC CATGCAAATG TTAAAACATA GCACTATAGA    60
   ACTCTGCAAG CCTCGAGTG                                                 79
(2) INFORMATION FOR SEQ ID NO: 19:
   (i) SEQUENCE CHARACTERISTICS:
      (A) LENGTH: 85 base pairs
      (B) TYPE: nucleic acid
      (C) STRANDEDNESS: single
      (D) TOPOLOGY: linear
   (ii) MOLECULE TYPE: genomic DNA
   (xi) SEQUENCE DESCRIPTION: SEQ ID NO: 19:
   GATCCTGCTA TGTCAGTTCC CCTTGGTTCT CTCATCTGGC CTGGTGCAAT AGGCCCTGCA    60
   TGCACTGGAT GCACTCTCAC TCGAG                                          85

## Description

The present invention relates to the field of molecular biology and provides methods and reagents which are particularly useful for the replication and amplification of ribonucleic acid (RNA) sequences. In another aspect, the invention provides methods for sterilizing a reverse transcription reaction, a homogeneous reverse transcription/amplification reaction or an amplification reaction contaminated with nucleic acids generated from a previous reverse transcription reaction. In a further aspect, the invention provides methods for reverse transcribing a target RNA molecule in a sample.

For a description of the related art and the explanation of the term used in the present patent application it is referred to a related patent application, viz. International Patent Application, Publication No. WO 91/09944, which provides high temperature reverse transcription methods.

In Abstract C-344, page 181, 94^{th} Meeting, May 23-27, Las Vegas, there is mentioned an buffer containing Mn²⁺ for RT-PCR with Thermus thermophilus DNA polymerase. The document is published after the priority date of the present application.

With regard to the present invention it is noted that Taq polymerase has been reported to inefficiently synthesize cDNA using Mg²⁺ as the divalent metal ion (Jones and Foulkes, 1989, Nuc. Acids. Res. 176:8387-8388). Tse and Forget, 1990, Gene 88:293-296; and Shaffer et al., 1990, Anal. Biochem. 190:292-296, have described methods for amplifying RNA using Taq polymerase and Mg²⁺ ion. However, the methods are inefficient and insensitive. For example, Tse and Forget demonstrate that 4 µg of total RNA is required to generate sufficient PCR product for ethidium bromide-stained gel visualization, using an abundantly expressed mRNA target. In addition, false positive signals from DNA template (PCR product from prior reactions or plasmid DNA contamination) were not rigorously excluded.

The present invention addresses this need and provides improved methods and reagents, in particular improved buffer systems for high temperature cDNA synthesis by thermoactive DNA polymerases. The reagents of the present invention are particularly suitable for a one enzyme, one tube, coupled reverse transcription/amplification assay using a thermostable DNA polymerase.

In one aspect, the invention provides a method for amplifying a target RNA molecule in a sample, the method comprising the steps of: (a) treating the sample in a reaction mixture comprising a first and second primer, wherein the first primer is sufficiently complementary to the target RNA to hybridize therewith and initiate synthesis of a cDNA molecule complementary to the target RNA, and the second primer is sufficiently homologous to the target RNA to hybridize to the cDNA and initiate synthesis of an extension product, and a thermostable DNA polymerase in the presence of all four deoxyribonucleoside triphosphates, in an appropriate buffer comprising a metal buffer which buffers the divalent cation concentration and which, in a preferred embodiment, buffers both the pH and the divalent cation concentration, wherein the reaction is performed at a temperature sufficient for the thermostable DNA polymerase to initiate synthesis of an extension product of the first primer to provide a cDNA molecule complementary to the target RNA; (b) treating the reaction mixture at an appropriate temperature to provide single-stranded cDNA; (c) treating the reaction mixture at an appropriate temperature for the thermostable DNA polymerase to initiate synthesis of an extension product of the second primer to provide a double-stranded cDNA molecule; and (d) amplifying the double-stranded cDNA molecule of step (c) by a polymerase chain reaction. Said appropriate buffers of steps (a) and (d) further comprise a buffering agent that binds said divalent cation, whereby said divalent cation is preferably Mn²⁺, wherein the K_{M} of the divalent cation binding reaction of said buffer at 20°C and 0.1M ionic strength is between 10 and 10⁶, preferably between 10² and 10⁴, more preferably between 10^{2.5} and 10^{3.5}. Said buffering agent is preferably a zwitterionic compound providing hydrogen ion buffering, wherein the pKa of said buffer at 20°C and 0.1M ionic strength is between 7 and 9, preferably between 7.5 and 8.5. In a preferred embodiment said buffer further comprises N,N-bis(2-hydroxyethyl)glycine or N[tris(hydroxymethyl)methyl]glycine, and more preferably further comprises an acetate salt selected from the group consisting of sodium acetate, potassium acetate, ammonium acetate and lithium acetate. In a most preferred embodiment, the buffer comprises manganese acetate (also written Mn(OAc)₂ or Mn(CH₃CO₂)₂), Bicine-KOH (Bicine is N,N-bis(2-hydroxyethyl)glycine), and potassium acetate (also written KOAc or KCH₃CO₂). Said thermostable DNA polymerase is preferably the Thermus aquaticus DNA polymerase or the Thermus thermophilus DNA polymerase, more preferably recombinant Tth DNA polymerase. In a preferred embodiment the temperature at step (a) is between 40°C and 80°C.

The present invention provides also methods for sterilizing reverse transcription reactions, amplification reactions, and homogeneous reverse transcription/amplification reactions, contaminated with nucleic acids generated from previous reverse transcription, amplification, and/or homogeneous reverse transcription/amplification reactions. For example the invention provides a method of sterilizing a reverse transcription reaction contaminated with nucleic acids generated from a previous reverse transcription wherein the previous reverse transcription resulted from mixing conventional and unconventional nucleoside triphosphates into a reverse transcription reaction mixture and generating cDNA products having the conventional and unconventional nucleotides incorporated therein, which method comprises degrading the contaminating nucleic acids by hydrolyzing covalent bonds of the unconventional nucleotides.

In another aspect, the invention provides a method of sterilizing a reverse transcription reaction contaminated with nucleic acids generated from a previous homogeneous reverse transcription/amplification reaction wherein the previous homogeneous reaction resulted from mixing conventional and unconventional nucleoside triphosphates into a homogeneous reverse transcription/amplification reaction mixture and generating cDNA and amplified products having the conventional and unconventional nucleotides incorporated therein, which method comprises degrading the contaminating amplified products by hydrolyzing covalent bonds of the unconventional nucleotides.

In one embodiment this method encompasses degrading the contaminating nucleic acid product with uracil-DNA glycosylase in an aqueous solution containing a target nucleic acid sequence; which further comprises inactivating the glycosylase in the presence of the target nucleic acid sequences (such as by heating); and, reverse transcribing and amplifying the target sequence by a thermostable DNA polymerase. The degradation of the contaminating product may be accomplished while the product is in contact with a nucleic acid reverse transcription/amplification reaction system. Thus, one can prepare a sample for reverse transcription/amplification, treat the sample by the present method to degrade any contaminating nucleic acid generated by a previous reverse transcription, amplification, and/or homogeneous reverse transcription/amplification reaction, and then amplify the target nucleic acid in the sample without having to adjust reaction volume or composition between steps.

In another aspect, the present invention provides reagents comprising a metal buffer which buffers the manganese ion concentration and which, in a preferred embodiment, buffers both the pH and the manganese ion concentration. The buffers significantly expand the usable range of manganese and dNTP concentrations in the methods of the present invention, thereby increasing the assay robustness and reducing problems with manganese chelators introduced during sample preparation. The buffers enable the use of higher dUTP concentrations in the sterilization methods of the present invention, which enhances dUTP incorporation in some targets and increases the efficiency of the sterilization by reducing the divalent metal ion concentration and lowering the ionic strength of the reaction mixture. The buffers also reduce the Mn²⁺ catalyzed RNA hydrolysis, which enables longer reverse transcription times for the reverse transcription of rare and/or longer targets. Furthermore, the buffers and reagents (e.g., dNTPs) of the present invention are easier to produce because of the relaxed concentration tolerances and provide improved storage and stability characteristics. Thus, the present invention provides a buffer for carrying out a homogeneous reverse transcription/amplification reaction comprising a divalent cation, a monovalent cation, and a buffering agent, wherein the divalent cation is Mn²⁺ and the buffering agent is a chelating agent that binds Mn²⁺, wherein the K_{M} of the manganese binding reaction of said buffering agent at 20°C and 0.1M ionic strength is between 10 and 10⁶, preferably between 10² and 10⁴, more preferably between 10^{2.5} and 10^{3.5}. In a preferred buffer said buffering agent is s zwitterionic compound providing hydrogen ion buffering, wherein the pKₐ if said buffer at 20°C and 0.1M ionic strength is between 7 and 9, preferably between 7.5 and 8.5. In a preferred embodiment of the invention the buffer comprises manganese acetate, Bicine-KOH, and potassium acetate. The present invention also provides a method for reverse transcribing a target RNA molecule in a sample, the method comprising the steps of: treating said sample in a reaction mixture comprising a primer, wherein said primer is sufficiently complementary to said target RNA to hybridize therewith and initiate synthesis of a cDNA molecule complementary to said target RNA, a thermoative DNA polymerase, four deoxyribonucleoside triphosphates, and an appropriate buffer, wherein said buffer comprises a divalent cation, preferably Mn²⁺, at a temperature sufficient for said thermoactive DNA polymerase to initiate synthesis of an extension product of said primer to provide a cDNA molecule complementary to said target RNA, wherein said buffer further comprises a buffering agent that binds Mn²⁺, wherein the K_{M} of the manganese binding reaction of said buffer at 20°C and 0.1M ionic strength is between 10 and 10⁶, preferably between 10² and 10⁴, more preferably between 10^{2.5} and 10^{3.5}.

For a detailed description of the background to the present invention it is referred to WO 91/09944 which is hereby incorporated by reference.

The following Figures may help to understand the present invention.

Figure 1 depicts the results of extension reactions described in Example 7, wherein the reaction efficiency is assayed over a range of manganese concentrations using different buffer conditions.

Figure 2 depicts the results of the RT/PCR described in Example 9, wherein the usable range of dNTP concentration is assayed using different buffer conditions.

The present invention relates to improved methods for efficiently reverse transcribing and amplifying RNA. Thus, e.g. methods are provided for a coupled, one tube procedure that eliminates the need to open the reaction vessel for modifying reaction components between the reverse transcription and amplification steps. In this way the effects of carryover contamination of RNA reverse transcription/amplification assays due to reverse transcribed or amplified products from previous reactions are minimized.

The methods of the present invention comprise treating a sample containing said RNA template with an oligonucleotide primer, which primer is sufficiently complementary to said RNA template to hybridize therewith, and a thermoactive DNA polymerase in the presence of all four deoxyribonucleoside triphosphates, in an appropriate buffer comprising a metal buffer which buffers the manganese ion concentration and which, in a preferred embodiment, buffers both the pH and the manganese ion concentration, and which reaction is performed at a temperature sufficient for said primer to hybridize to said RNA template and said thermoactive DNA polymerase to catalyze the polymerization of said deoxyribonucleoside triphosphates to form a cDNA sequence complementary to the sequence of said RNA template. According to the invention, the DNA polymerase may be thermostable as well as thermoactive.

In another aspect, a primer suitable for annealing to an RNA template may also be suitable for amplification by PCR. For PCR, a second primer, complementary to the reverse transcribed cDNA strand, provides a site for initiation of synthesis of an extension product.

In the amplification of an RNA molecule by a thermoactive DNA polymerase, the first extension reaction is reverse transcription using an RNA template, and a DNA strand is produced. The second extension reaction, using the DNA template, produces a double-stranded DNA molecule. Thus, synthesis of a complementary DNA strand from an RNA template by a thermoactive DNA polymerase provides the starting material for amplification.

Thermostable DNA polymerases can be used in a coupled, one-enzyme reverse transcription/amplification reaction. Methods are provided for both non-homogeneous and homogeneous RT/PCR assays. The term "homogeneous" as used herein refers to a two-step single addition reaction for reverse transcription and amplification of an RNA target. By homogeneous it is meant that following the reverse transcription step, there is no need to open the reaction vessel or otherwise adjust reaction components prior to the amplification step. In a non-homogeneous RT/PCR reaction, following reverse transcription and prior to amplification any one or more of the reaction components is adjusted, added, or diluted including enzyme, primers, divalent cation, salts, pH, or dNTPs.

The term "homogeneous reverse transcription/amplification reaction mixture" refers to an aqueous solution comprising the various reagents used to reverse transcribe and amplify a target RNA. These include enzymes, aqueous buffers, salts, oligonucleotide primers, target nucleic acid, and nucleoside triphosphates. Depending upon the context, the mixture can be either a complete or incomplete homogeneous reverse transcription/amplification reaction mixture.

The present invention relates also to simplified and improved methods for detecting RNA target molecules in a sample. These methods employ thermostable DNA polymerases to catalyze reverse transcription, second strand cDNA synthesis, and, if desired, amplification. Prior methods required two sets of incubation conditions, necessitated by the use of different enzymes for each procedure. The methods of the present invention provide RNA transcription and amplification with significantly enhanced specificity and with fewer steps than previous RNA cloning and diagnostic methods. These methods are adaptable for use in kits for laboratory or clinical analysis.

The term "reverse transcription reaction mixture" refers to an aqueous solution comprising the various reagents used to reverse transcribe a target RNA. These include enzymes, aqueous buffers, salts, oligonucleotide primers, target nucleic acid, and nucleoside triphosphates. Depending upon the context, the mixture can be either a complete or incomplete reverse transcription reaction mixture.

For amplification of the cDNA product a number of methods are available to one of ordinary skill in the art. As used herein the term "amplification reaction system" refers to any in vitro means for multiplying the copies of a target sequence of nucleic acid. Such methods include but are not limited to polymerase (PCR), DNA ligase (LCR), Qβ RNA replicase, and RNA transcription-based (TAS and 3SR) amplification systems. However, the homogeneous RT/PCR methods described herein have significant advantages over multistep, multi-enzyme amplification methods such as 3SR in that only one polymerase enzyme is used.

Indeed, several different nucleic acid-based amplification systems can benefit from the considerable broadening of the divalent metal ion (e.g., Mn²⁺) optima, and the surprising expansion of the distinctly different dual optima for RNA template-directed synthesis and DNA template-directed synthesis. All of these systems are based on the PCR process in that a product synthesized in one cycle or in a portion of a reaction serves as template in a succeeding cycle or in a succeeding portion of a reaction. In the Ligase Chain Reaction (LCR, Wu and Wallace, 1989, Genomics 4:560-569 and Barany, 1991, Proc. Natl. Acad. Sci. USA 88:189-193) , four oligonucleotides are used to amplify an intended segment of DNA with a DNA ligase in the presence of Mg²⁺ and necessary cofactors. Use of stringent annealing temperatures and a thermoactive and thermoresistant DNA ligase significantly improves the sensitivity of this process and the ability to discriminate allelic differences. Whether one uses a native thermophilic DNA ligase (e.g., Takahashi, Yamaguchi and Uchida, 1984, J. Biol. Chem. 259:10041-10047, incorporated herein by reference) or a recombinant thermophilic DNA ligase (e.g., Barany and Gelfand, 1991, Gene 109:1-11), the buffers and buffering agents of this invention are useful for broadening the metal ion optima, for facilitating both RNA template-directed ligation and DNA template-directed ligation, and for increasing the stability of RNA templates at elevated temperatures in the presence of divalent metal ions.

Similarly, nucleic acid amplification processes derived from PCR and LCR which use two or more enzymes (e.g., Polymerase Ligase Chain Reaction, Barany, 1991, PCR Methods and Applic. 1:5-16; or Gap-LCR, PCT Patent Publication No. WO 90/01069; or Repair Chain Reaction, European Patent Application, Publication No. EP-A-439,182 will benefit from the buffers and buffering agents of the present invention due to the broadening of the divalent metal ion optima for either or both RNA template-directed synthesis and DNA template-directed synthesis and to the increased stability of RNA templates at elevated temperatures in the presence of divalent metal ions. This is of particular advantage when the different nucleic acid interacting enzymes used in the process normally have different divalent metal ion optima.

Similarly, isothermal nucleic acid amplification processes (e.g., 3SR, Kwoh et al., 1989, Proc. Natl. Acad. Sci. USA 86:1173-1177, Guatelli et al., 1990, Proc. Natl. Acad. Sci. USA 87:1874-1878, PCT Patent Application, Publication No. WO 92/0880A; NASBA, U.S. Patent No. 5,130,238) that utilize multiple enzymes will benefit from this invention and the broadening of the divalent metal ion optima for either or both RNA template-directed synthesis and DNA template-directed synthesis. This is of particular advantage when the different nucleic acid interacting enzymes used in the process normally have different divalent metal ion optima. The isothermal amplification systems described above all utilize mesophilic or nonthermoactive and thermosensitive nucleic acid binding enzymes. Isothermal amplifications systems based on analogous thermoactive and thermoresistant enzymes (e.g., substitution of [1] Thermus thermophilus DNA polymerase for AMV or MoMuLV reverse transcriptase, [2] Thermus thermophilus RNase H [Itaya and Kondo, 1991, Nuc. Acids Res. 19:4443-4449] for E. coli RNase H and [3] Thermus thermophilus phage φYS40 [Sakaki and Oshima, 1975, J. Virol. 15:1449-1453] RNA polymerase for bacteriophage T3, T7 or SP6 RNA polymerases) will particularly benefit from the buffers and buffering agents of the present invention for the reasons stated above as well as the increased stability at elevated temperatures of RNA templates in the presence of divalent metal ions and the buffers and buffering agents of this invention.

This invention is not limited to any particular amplification system. As other systems are developed, those systems may benefit by practice of this invention. A recent survey of amplification systems was published in Abramson and Myers, 1993, Current Opinion in Biotechnology 4:41-47.

The term "amplification reaction mixture" refers to an aqueous solution comprising the various reagents used to amplify a target nucleic acid. These include enzymes, aqueous buffers, salts, amplification primers, target nucleic acid, and nucleoside triphosphates. Depending upon the context, the mixture can be either a complete or incomplete amplification reaction mixture. In the preferred embodiment of the invention the amplification system is PCR and the amplification reaction mixture is a PCR mixture.

The term "buffer", as used herein, refers to a solution containing a buffering agent or a mixture of buffering agents and, optionally, a divalent cation and a monovalent cation.

The present invention is suitable for transcribing and amplifying RNA from a number of sources. The RNA template may be contained within a nucleic acid preparation from an organism, for example, a viral or bacterial nucleic acid preparation. The preparation may contain cell debris and other components, purified total RNA, or purified mRNA. The RNA template may be a population of heterogeneous RNA molecules in a sample or a specific target RNA molecule.

RNA suitable for use in the present methods may be contained in a biological sample suspected of containing a specific target RNA. The biological sample may be a heterogeneous sample in which RNA is a small portion of the sample, as in for example, a blood sample or a biopsied tissue sample. Thus, the method is useful for clinical detection and diagnosis. The RNA target may be indicative of a specific disease or infectious agent.

RNA is prepared by any number of methods such as those referred to in WO 91/09944.

Synthetic oligonucleotides can be prepared using the triester method of Matteucci et al., 1981, J. Am. Chem. Soc. 103:3185-3191. Alternatively automated synthesis may be preferred, for example, on a Biosearch 8700 DNA Synthesizer using cyanoethyl phosphoramidite chemistry.

For primer extension to occur this primer must anneal to the RNA template. Not every nucleotide of the primer must anneal to the template for reverse transcription to occur. The primer sequence need not reflect the exact sequence of the template. For example, a non-complementary nucleotide segment may be attached to the 5' end of the primer with the remainder of the primer sequence being complementary to the RNA. Alternatively, non-complementary bases can be interspersed into the primer, provided that the primer sequence has sufficient complementarity with the RNA template for hybridization to occur and allow synthesis of a complementary DNA strand.

Prior methods of cDNA preparation required a pre-annealing step. Destabilization of secondary and tertiary structure of the RNA template may be required to allow the primer to hybridize to the RNA. Generally, annealing is accomplished by various means and is routinely accomplished in the presence of an annealing buffer. Maniatis et al., 1982, "Molecular Cloning: A Laboratory Manual", Cold Spring Harbor, New York, provide examples of annealing buffers. Annealing methods include, but are not limited to, incubating the RNA/primer mixture at a high temperature for a short period of time followed by step-wise cooling or quick chilling the mixture in a dry ice/ethanol bath. To prevent intrastrand secondary structure interactions from interfering with cDNA synthesis or primer annealing, at the low temperatures used previously for reverse transcription, some investigators modify the RNA template by treatment with chemical denaturants such as methylmercury hydroxide (Baily and Davidson, 1976, Anal. Biochem. 70:75). However, such denaturants are generally highly toxic, carcinogenic compounds and must be carefully removed to prevent enzyme inhibition.

Although the primer must anneal to the template for reverse transcription to occur, a separate annealing step is not a necessity. Because thermoactive reverse transcriptase activity is not irreversibly denatured at the high temperatures preferred for stringent annealing, there is no need for the quick chill or step-wise cooling of the denatured template, prior to the addition of the polymerase. Prior methods necessitated that the heated, denatured RNA was cooled in a manner that would maintain the annealed primer-template structure while reducing the temperature to provide conditions compatible with enzyme activity, usually 37-42°C. Methods for high temperature reverse transcription of RNA and eliminates the necessity of a pre-annealing step and the use of chemical denaturants (see e.g. Examples 1 to 4). The present invention provides improved means for destabilizing RNA secondary and tertiary structure. The elevated temperatures for reverse transcription with a thermostable DNA polymerase further destabilize RNA secondary and tertiary structure and also serves to denature double-stranded RNA target.

The present methods provide that reverse transcription of the annealed primer-RNA template is catalyzed by a thermoactive or thermostable DNA polymerase. As used herein, the term "thermostable polymerase" refers to an enzyme that is heat stable or heat resistant and catalyzes polymerization of deoxyribonucleotides to form primer extension products that are complementary to a nucleic acid strand. Thermostable DNA polymerases useful herein are not irreversibly inactivated when subjected to elevated temperatures for the time necessary to effect destabilization of single-stranded nucleic acids or denaturation of double-stranded nucleic acids during PCR amplification. Irreversible denaturation of the enzyme refers to substantial loss of enzyme activity. Preferably a thermostable DNA polymerase will not irreversibly denature at about 90-100°C under polymerization conditions.

In another aspect of the invention, it is only essential that the DNA polymerase for high temperature reverse transcription is thermoactive. As used herein, the term "thermoactive polymerase" refers to an enzyme that is capable of efficiently catalyzing polymerization of deoxyribonucleotides to form a primer extension product complementary to a nucleic acid template strand at temperatures equal to or greater than 60°C. According to the present invention, thermoactive polymerases for reverse transcription have maximal activity over 50°C. The thermoactive DNA polymerase will not irreversibly denature at temperatures between 50-80°C under conditions for RNA destabilization and primer annealing.

In the examples provided, the thermoactive DNA polymerases are also thermostable; however, a thermoactive, non-thermostable enzyme is also suitable for practicing the present invention. Because the preparation of cDNA from an RNA template does not involve repeated denaturation cycles at elevated temperatures, it is not essential that enzymes useful in the method are thermostable as well as thermoactive. However, in one embodiment of the invention, a homogeneous RT/PCR procedure is provided. Because the reaction components are not adjusted between the RT and PCR steps, a thermostable DNA polymerase is preferred.

The heating conditions will depend on the buffer, salt concentration, and nucleic acids being denatured. Of course, it will be recognized that for the reverse transcription of mRNA, the template molecule is generally single-stranded and, therefore, a high temperature denaturation step is unnecessary. However, double-stranded RNA also provides a suitable template for the reverse transcription/amplification methods described, following an initial denaturation or strand-separation step. The present invention provides buffers which decrease RNA degradation at the high temperatures required for heat denaturation of double-stranded RNA, thereby improving the efficiency of reverse transcription/amplification reactions from double-stranded RNA templates. The buffers of the present invention permit brief, very high temperature treatment of the- complete reaction mixture to further destabilize any RNA secondary and tertiary structure immediately prior to the primer annealing step of the reverse transcription reaction (Example 12). Double-stranded RNA templates may include, for example, Reo virus, blue tongue virus, Colorado tick fever virus, and yeast killer factor.

Temperatures for RNA destabilization typically range from 50-80°C. A first cycle of primer elongation provides a double-stranded template suitable for denaturation and amplification as referred to above. Temperatures for nucleic acid denaturation typically range from about 90 to about 100°C for a time sufficient for denaturation to occur, which depends on the nucleic acid length, base content, and complementarity between single-strand sequences present in the sample, but typically about 10 seconds to 4 minutes.

The thermostable or thermoactive DNA polymerase preferably has optimum activity at a temperature higher than about 40°C, e.g., 60-80°C. At temperatures much above 42°C, DNA and RNA-dependent polymerases, other than thermostable or thermoactive DNA polymerases, are inactivated. Shimomave and Salvato, 1989, Gene Anal. Techn. 6:25-28, describe that at 42°C AMV-RT has maximum activity. At 50°C the enzyme has 50% activity, and at 55°C AMV-RT retains only 10% of its optimal level of activity. Thus, AMV-RT is inappropriate for catalyzing high temperature polymerization reactions utilizing an RNA template. Only the present method provides methods for efficient high temperature reverse transcription with thermoactive DNA polymerases.

Hybridization of primer to template depends on salt concentration as well as composition and length of primer. When using a thermostable or thermoactive polymerase, hybridization can occur at higher temperatures (e.g., 45-70°C) which are preferred for increased selectively and/or higher stringency of priming. Higher temperature optimums for the polymerase enzyme enable RNA reverse transcription and subsequent amplification to proceed with greater specificity due to the selectivity of the primer hybridization process. Preferably, the optimum temperature for reverse transcription of RNA ranges from about 55-75°C, more preferably 60-70°C.

The present invention provides a method for reverse transcription of an RNA template, having enhanced primer-directed specificity, catalyzed by a thermostable DNA polymerase. The methods disclosed are improved over prior methods for the reverse transcription of RNA. These methods provide for the amplification of an RNA segment via an RNA/cDNA hybrid intermediate molecule. The hybrid molecule is a suitable template for amplification by PCR. Thus, the reverse transcription and amplification reactions are coupled. Previous RNA amplification methods require incubation of the RNA/primer mixture in the presence of reverse transcriptase at 37-42°C prior to the initiation of an amplification reaction. Only by the present invention are all of the enzymatic steps for RNA amplification catalyzed by a thermostable DNA polymerase. The advantages brought to PCR by the commercial availability of Taq and Tth DNA polymerases, the disclosed methods for preparing Tth DNA polymerase, and the commercial availability of Tth DNA polymerase reverse transcription/DNA amplification kits (Perkin Elmer, Norwalk, CT) are now, by the methods disclosed herein, applicable to reverse transcription, RNA detection, cDNA preparation and coupled reverse transcription/cDNA amplification of RNA.

DNA amplification procedures by PCR are well known and are described in U.S. Patent Nos. 4,683,195, 4,683,202, and 4,965,188.

For ease of understanding the advantages provided by the present invention, a summary of PCR is provided. PCR requires two primers that hybridize with the double-stranded target nucleic acid sequence to be amplified. In PCR, this double-stranded target sequence is denatured and one primer is annealed to each strand of the denatured target. The primers anneal to the target nucleic acid at sites removed from one another and in orientations such that the extension product of one primer, when separated from its complement, can hybridize to the other primer. Once a given primer hybridizes to the target sequence, the primer is extended by the action of a DNA polymerase. The extension product is then denatured from the target sequence, and the process is repeated.

In successive cycles of this process, the extension products produced in earlier cycles serve as templates for DNA synthesis. Beginning in the second cycle, the product of amplification begins to accumulate at a logarithmic rate. The amplification product is a discrete double-stranded DNA molecule comprising: a first strand which contains the sequence of the first primer, eventually followed by the sequence complementary to the second primer, and a second strand which is complementary to the first strand.

Due to the enormous amplification possible with the PCR process, small levels of DNA carryover from samples with high DNA levels, positive control templates or from previous amplifications can result in PCR product, even in the absence of purposefully added template DNA. If possible, all reaction mixes are set up in an area separate from PCR product analysis and sample preparation. The use of dedicated or disposable vessels, solutions, and pipettes (preferably positive displacement pipettes) for RNA/DNA preparation, reaction mixing, and sample analysis will minimize cross contamination. See also Higuchi and Kwok, 1989, Nature, 339:237-238 and Kwok, and Orrego, in: Innis et al. eds., 1990 "PCR Protocols: A Guide to Methods and Applications", Academic Press, Inc., San Diego.

One particular method for minimizing the effects of cross-contamination of nucleic acid amplification is described in PCT Patent Publication No. WO 92/01814 and U.S. Patent No. 5,035,996, The method involves the introduction of unconventional nucleotide bases, such as dUTP, into the amplified product and exposing carryover product to enzymatic and/or physical-chemical treatment to render the product DNA incapable of serving as a template for subsequent amplifications. For example, uracil-DNA glycosylase, also known as uracil-N-glycosylase or UNG, will remove uracil residues from PCR product containing that base. The enzyme treatment results in degradation of the contaminating carryover PCR product and serves to "sterilize" the amplification reaction.

The term "unconventional" when referring to a nucleic acid base, nucleoside, or nucleotide includes modifications, derivations, or analogs of conventional bases, nucleosides or nucleotides which naturally occur in a particular polynucleotide (e.g., DNA [dA, dG, dC, dT] or RNA [A, G, C, U]). Uracil is a conventional base in RNA (i.e., covalent attachment to ribose in a ribopolynucleotide) but an unconventional base in DNA (i.e., covalent attachment to deoxyribose in a deoxyribopolynucleotide). In a coupled RT/PCR reaction, it is desirable to sterilize the reaction prior to the RT step to eliminate carryover nucleic acid products of prior reverse transcription and/or amplification reactions. Sterilization after reverse transcription, and prior to PCR, results in degradation of noncontaminating cDNA products containing dUTP, as well as contaminating product. Synthesis of cDNA in the presence of dTTP and absence of dUTP is impractical. For efficient incorporation of dUTP into the subsequent PCR product, a vast excess of dUTP would be required to dilute the dTTP present as carryover from the reverse transcription step. Furthermore, this would require opening the tube in order to add the dUTP. Consequently, the effectiveness of UNG sterilization would be diminished.

The present invention provides methods for sterilization of the RT/PCR reaction. Example 4 demonstrates this aspect of the invention. When unconventional nucleosides are being incorporated into amplification products, routine titration experiments are useful to optimize reaction conditions, and European Patent Application EP-A-540,693, provides guidance for incorporating unconventional nucleotides. The parameters which are varied include, but are not limited to the concentration of divalent cation, pH range, concentration of DNA polymerase, concentration of the unconventional nucleoside, the addition of natural nucleoside for which the unconventional nucleoside is inserted, time of each cycle, and temperature.

Generally, the concentration of dNTPs in a PCR using MgCl₂ in a Tris buffer is within the range 20-200 µM for each dNTP. For incorporating dUTP the efficiency of amplification is improved at an elevated nucleotide concentration. In Example 4, the concentration of dUTP in the PCR is 200 µM and dCTP, dGTP, and dATP are also present at the same concentration, although this is not essential. When the concentration of dUTP or dNTPs is increased, the concentration of MgCl₂ and MnCl₂ is increased accordingly. In Example 4, the PCR contains 200 µM of each dGTP, dATP, dUTP, and dCTP and 2 mM MgCl₂, and provides efficient amplification.

For reverse transcription using a thermostable polymerase, Mn²⁺ is preferred as the divalent cation and is typically included as a salt, for example, manganese chloride (MnCl₂), manganese acetate (Mn(OAc)₂), or manganese sulfate (MnSO₄). If MnCl₂ is included in a reaction containing 10 mM Tris buffer, for example, the MnCl₂ is generally present at a concentration of 0.5-7.0 mM; 0.8-1.4 mM is preferred when 200 µM of each dGTP, dATP, dUTP, and, dCTP are utilized; and 1.2 mM MnCl₂ is most preferred. The present invention provides methods and reagents to expand the usable range of the divalent cation concentration. In one embodiment of the invention, a reaction buffer comprising Mn(OAc)₂, Bicine-KOH, and KOAc is used in place of the MnCl₂, Tris, KCl buffer. Example 8 describes the use of such a bicine/acetate buffer in which the Mn²⁺, supplied as Mn(OAc)₂, is used at concentrations ranging from 1.2 to 2.5 mM; concentrations of 3.6 mM and 3.5 mM are utilized in the RT/PCR described in Example 10.

The optimal concentrations of the unconventional nucleotide and divalent cation may vary in the reverse transcription reaction, just as for the amplification reaction as noted above, depending on the total dNTP concentration and on the particular primers, template, buffer, salts, and polymerase present. The examples describe the use of a bicine/acetate buffer which allows a higher concentration of dUTP in the reverse transcription reaction, thereby enhancing the incorporation of dUMP into newly synthesized cDNA.

In one embodiment of the invention, described in Example 3, a two step, single addition procedure is provided for coupled RT/PCR using MnCl₂ as the divalent cation in a Tris-HCl buffer for both the RT and PCR steps. In the method, following reverse transcription, no buffer adjustment is made prior to the PCR step. Consequently, a lower concentration of MnCl₂ is used, whether incorporating dTTP or dUTP (using 200 µM dNTPs), to avoid a reduction in amplification efficiency that may occur when MnCl₂ concentration is maintained at 1.2 mM during PCR.

Following amplification and analysis of the RT/PCR result, the RT/PCR product may be introduced unintentionally as a contaminant in other reactions. Prior to subsequent RT, RT/PCR or amplification reactions, the reaction mixtures are treated with a DNA glycosylase specific for the unconventional nucleotide incorporated during the prior RT/PCR. In this manner, any previous RT/PCR product, present as a contaminant in subsequent RT, RT/PCR or amplification reaction mix containing a target nucleic acid, is hydrolyzed.

Consequently, in practice, the sterilization treatment is carried out prior to the RT/PCR assay to eliminate carryover of dUMP containing product DNAs. For example, prior to the high temperature (60-70°C) incubation of the reverse transcription reaction mix, 0.01-0.05 units UNG per µl of RT reaction volume is added and incubated for 1-10 minutes at room temperature. Alternatively, the incubation is carried out for 2 minutes at 50°C. The subsequent high temperature (60-70°C) RT and 95°C denaturation steps serve to inactivate UNG so that newly synthesized cDNA and PCR products are not degraded. UNG is commercially available from Perkin Elmer, Norwalk, CT. EP-A-540,693, describes methods for producing UNG by recombinant means and also thermolabile UNG derivatives which do not regain activity after heating above the denaturation temperature of the DNA sample. Such derivatives may be preferred for practicing the present invention.

The target of amplification can be an RNA/DNA hybrid molecule. The target can be a single-stranded or double-stranded nucleic acid. Although the PCR procedure described above assumed a double-stranded target, this is not a necessity. After the first amplification cycle of a single-stranded DNA target, the reaction mixture contains a double-stranded DNA molecule consisting of the single-stranded target and a newly synthesized complementary strand. Similarly, following the first amplification cycle of an RNA/cDNA target, the reaction mixture contains a double-stranded cDNA molecule. At this point, successive cycles of amplification proceed as described above. In the present methods, the target of amplification is a single-stranded RNA, and the first amplification cycle is the reverse transcription step. Alternatively, if the starting template is double-stranded RNA, an initial high temperature denaturing step may be used to prepare single-stranded RNA template.

As used herein the term "cDNA" refers to a complementary DNA molecule synthesized using a ribonucleic acid strand (RNA) as a template. The RNA may be mRNA, tRNA, rRNA, or another form of RNA, such as viral RNA. The cDNA may be single-stranded, double-stranded or may be hydrogen-bonded to a complementary RNA molecule as in an RNA/cDNA hybrid.

The methods of the present invention provide means for obtaining cDNA from a desired RNA template wherein the desired end product is produced with greater specificity than by previous methods. Additionally, the present invention provides that cDNA synthesis can be coupled to amplification by PCR. These methods incorporate previously unknown properties of thermoactive DNA polymerases. In the disclosed embodiments, methods are provided which utilize Taq and Tth DNA polymerases for reverse transcription. These embodiments should not be construed as a limitation of the present invention.

Thermostable polymerases are available from a number of sources. The enzyme may be a native or recombinant protein. A preferred thermostable enzyme is Thermus thermophilus DNA polymerase (Tth DNA polymerase), purified from Thermus thermophilus (see PCT Patent Application Publication No. WO 91/09950.

Alternatively, Tth DNA polymerase is purified from recombinant host cells, which host cells may be prepared as described in WO 91/09944 using the primers

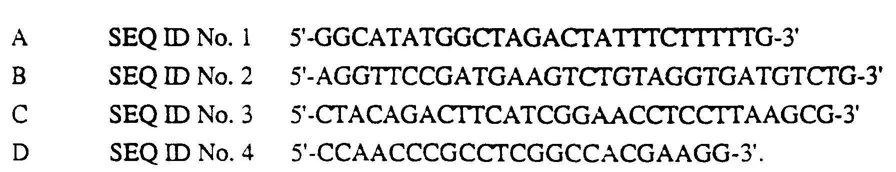

The recombinant Tth polymerase is designated rTth DNA polymerase. Also preferred for practicing the invention is Taq DNA polymerase. Taq DNA polymerase is commercially available as a recombinant product or purified as native Taq DNA polymerase from Thermus aquaticus developed and manufactured by Hoffmann-La Roche Inc. and commercially available from Perkin Elmer, Norwalk, CT. As used herein, recombinant Taq DNA polymerase may be designated as rTaq DNA polymerase and native Taq DNA polymerase may be designated as nTaq DNA polymerase. In addition, T. flavus (Tfl) DNA polymerase, available from Amersham, Arlington Heights, IL, as "Hot Tub" DNA polymerase may be suitable. Further details on the use of Tth DNA polymerase for reverse transcription and amplification of nucleic acids can be found in WO 91/09944.

Primers can be designed with convenient restriction enzyme recognition sequences located at or near the 5' end of the primer. In the formation of the cDNA transcript, so long as the 3' end of the primer is hydrogen-bonded to the target sequence, the resulting double-stranded cDNA molecule would contain a specific restriction enzyme recognition sequence. Following amplification, using the cDNA as a template, the restriction site could be used to facilitate other procedures, for example, cloning.

Following reverse transcription of RNA by a thermoactive or thermostable DNA polymerase, the RNA can be removed from the RNA/cDNA hybrid by heat denaturation or by a number of other known means such as alkali, heat, or enzyme treatment. Enzyme treatment may consist of, for example, treating the RNA/cDNA hybrid with RNase H. RNase H is specific for RNA strands within an RNA/DNA double-stranded molecule. Tth and Taq associated RNase H and 5'→3' nuclease activities can facilitate hydrolysis of the RNA template and synthesis of the second DNA strand, as well as primer extension for amplification of the cDNA sequence. Alternatively, exogenous RNase H is added from a commercially available source.

The RNase H activity of thermostable polymerases provides means for distinguishing between RNA and DNA templates in a sample. This is particularly useful for detecting RNA in the presence of homologous duplex DNA. Where the DNA is free of introns in, for example, proviral HIV DNA, amplified RNA, and DNA may not be distinguishable by size. However, following reverse transcription, thermostable RNase H activity eliminates the necessity for denaturating the RNA/cDNA duplex. Consequently, in the presence of genomic or proviral DNA, only the RNA template is amplified in the first PCR cycle.

In a preferred method for distinguishing between homologous RNA and DNA templates, amplification primers are used to direct the synthesis of the PCR product with a low strand separation temperature. For example, for detecting HIV RNA, primer pairs:

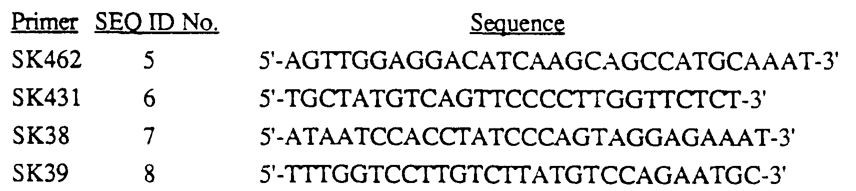

generate a PCR product that is denatured well below 94°C. Typical denaturation temperatures for PCR are 94-96°C. At the lowered temperature the double-stranded DNA (i.e., the expected "contaminant") is not denatured and would not be amplified. Methods for affecting the denaturation temperature of PCR products are described in detail in European Patent Application EP-A-519,338.

Alternatively, unconventional nucleotides are useful for effecting the denaturation temperature of the PCR product. For example, hydroxymethyl dUTP (HmdUTP) naturally occurs in SP01 phage DNA as 5' hydroxymethyluracil (HmUra) in place of thymine (Kallen et al., 1962, J. Mol. Biol. 5:248-250, and Levy and Teebor, 1991, Nuc. Acids Res. 19(12):3337. The HmUra containing genome melts at 10°C less than DNA of corresponding thymine content. Incorporation of HmdUMP into cDNA effectively lowers the denaturation temperature of both the reverse transcribed product and the PCR duplex DNA product, in comparison to the denaturation temperature of the homologous thymine containing DNA. Other modified nucleoside triphosphates capable of effecting the Tm of the DNA product (e.g., c⁷dGTP, 7 deaza-2'deoxyguanosine 5'-triphosphate or α-phosphorothioate dNTPs) are also suitable for distinguishing between homologous RNA and DNA templates.

Following removal or melting of the RNA template strand, the remaining cDNA strand can then serve as a template for polymerization of a self-complementary strand, providing a double-stranded cDNA molecule suitable for additional amplification, detection or other manipulation. The second strand synthesis also requires a primer. A sequence specific primer can be introduced into the reaction mix to prime second strand synthesis. Alternatively, a duplex adapter-linker may be ligated to the cDNA or the cDNA may be tailed with a terminal transferase-type activity. The second strand primer needs only to hybridize to the tail rather than the specific cDNA sequence (see for example, Frohman in Innis et al., supra). In the practice of the disclosed methods, it may be desirable to use a first set of primers to synthesize a specific cDNA molecule and a second nested set of primers to amplify a desired cDNA segment. All of these reactions may be catalyzed by the same thermostable DNA polymerase.

For reverse transcription, according to the present invention, the primer-template mixture is incubated with a thermoactive or thermostable polymerase under suitable polymerization conditions. These conditions are provided by a reaction mixture containing all four deoxyribonucleotide triphosphates (dNTPs) and a buffer containing a buffering agent, a divalent cation, and a monovalent cation.

DNA polymerases require a divalent cation for catalytic activity. Tabor and Richardson, 1989, Proc. Natl. Acad. Sci. USA 86:4076-4080, have reported that Mn²⁺ can be substituted for Mg²⁺ in DNA sequencing methods. These methods require a DNA template and T7 DNA polymerase or DNA polymerase I.

Either Mn²⁺, Mg²⁺, or Co²⁺ can activate Taq, Tth. and Tma DNA polymerases; however, Mn²⁺ is preferred in the present methods. In the disclosed embodiments of the invention, buffers are provided which contain Mn²⁺ for nucleic acid reverse transcription from an RNA template. These buffers are improved over previous reverse transcription buffers and result in increased cDNA yields. In particular, practice of the present methods using Tth DNA polymerase and MnCl₂ or Mn(OAc)₂ for amplifying RNA imparts an increase in sensitivity of at least 10⁶-fold compared to MgCl₂ and standard PCR conditions. While capable of activating RNA template-directed DNA synthesis, mixed divalent cation buffers (e.g., Mn²⁺ and Mg²⁺), are not preferred due to reduced sensitivity and efficiency.

The divalent cation is supplied in the form of a salt such MgCl₂, Mg(OAc)₂, MgSO₄, MnCl₂, Mn(OAc)₂, or MnSO₄. Usable cation concentrations in a Tris-HCl buffer are for MnCl₂ from 0.5 to 7 mM, preferably, between 0.5 and 2 mM, and for MgCl₂ from 0.5 to 10 mM. Usable cation concentrations in a Bicine/KOAc buffer are from 1 to 20 mM for Mn(OAc)₂, preferably between 2 and 5 mM.

Preferably, the monovalent cation is supplied by the potassium, sodium, ammonium, or lithium salts of either chloride or acetate. For KCl, the concentration is between 1 and 200 mM, preferably the concentration is between 40 and 100 mM, although the optimum concentration may vary depending on the polymerase used in the reaction. Optimal reverse transcriptase activity is observed between 50 and 75 mM KCl when Tth DNA polymerase is used. However, enhanced RT/PCR is observed when the KCl concentration is increased up to 100 mM. For a Taq DNA polymerase such as AmpliTaq® DNA polymerase, 50 mM KCl is preferred. For KOAc, optimal reverse transcriptase activity is observed at concentrations between 85 mM and 115 mM when Tth DNA polymerase is used.

Deoxyribonucleotide triphosphates are added as solutions of the salts of dATP, dCTP, dGTP, dUTP, and dTTP, such as disodium or lithium salts. In the present methods, a final concentration in the range of 1 µM to 2 mM each is suitable, and 100-600 µM is preferable, although the optimal concentration of the nucleotides may vary in the reverse transcription reaction depending on the total dNTP and divalent metal ion concentration, and on the buffer, salts, particular primers, and template. For longer products, i.e., greater than 1500 bp, 500 µM each dNTP and 2 mM MnCl₂ may be preferred when using a Tris-HCl buffer.

A suitable buffering agent is a zwitterionic compound providing hydrogen ion buffering and preferably buffers both the pH and the manganese ion concentration as specified in the first paragraph on page 2. A specially preferred buffering agent is Bicine-KOH, preferably pH 8.3, although pH may be in the range 7.8-8.7. Bicine acts both as a pH buffer and as a metal buffer.

Additionally, EDTA less than 0.5 mM may be present in the reverse transcription reaction mix. Detergents such as Tween-20™ and Nonidet™ P-40 are present in the enzyme dilution buffers. A final concentration of non-ionic detergent approximately 0.1% or less is appropriate, however, 0.01-0.05% is preferred and will not interfere with polymerase activity. Similarly, glycerol is often present in enzyme preparations and is generally diluted to a concentration of 1-20% in the reaction mix. A mineral oil overlay may be added to prevent evaporation but is not necessary when using a TC9600 thermal cycler (Perkin Elmer, Norwalk, CT) or when using Ampliwax™ PCR Gem 100 (Perkin Elmer, Norwalk, CT) as described in PCT Patent Publication No. WO 91/12342 and Chou et al., 1992, Nuc. Acids. Res. 20:1717-1723,

In one embodiment of the invention, a method for homogeneous RT/PCR is provided. This two-step, single addition procedure eliminates the need to open the tube after the addition of initial reagents. Thus, the opportunity for contamination between the RT and PCR steps is removed. However, the opportunity to change reaction reagents between steps is also eliminated and the two reactions need to be performed under the same reaction reagent conditions, which may not be optimal for both the RT and PCR steps. Adjustment of reaction conditions may be required to accommodate the separate requirements of the two reaction steps. For example, due to the high enzyme concentration required for optimum RT activity, a short extension cycle is preferred, i.e., 10-30 seconds, during each PCR thermal Cycler when amplifying short targets, although the extension cycle time may depend on the thermal cycler used. For example 1 minute is preferred when using a TC480 thermal cycler marketed by Perkin Elmer, Norwalk, CT.

Similarly, because there is no buffer adjustment between the RT and PCR steps in a homogeneous RT/PCR assay, a manganese concentration intermediate to the separate RT and PCR optima is required such that each step functions. As shown in Example 7, the optimal concentration is lower for the DNA target, PCR reaction than it is for the RNA target, RT reaction. The manganese concentration providing optimal synthesis on DNA templates was found to be approximately 0.6 mM (with 800 µM total dNTP, Tris buffer), the enzyme obtained maximal reverse transcriptase activity on the RNA template at approximately 1.4 mM manganese (with 800 µM total dNTP, Tris buffer). For the homogeneous reaction described in Example 3, the MnCl₂ concentration is preferably equal to or less than 1.0 mM and, most preferably, 0.8 mM. While the preferred concentrations represent conditions which allow Tth DNA polymerase to perform RT/PCR, these conditions are suboptimal for both reverse transcription and DNA amplification. Furthermore, although the efficiency of the RT step in the presence of dUTP, as in the sterilization methods of the present invention, is improved at higher MnCl₂ concentration, the PCR step is less efficient. Consequently, 0.8 mM MnCl₂ most preferably is used and the product is detected by means more sensitive than ethidium stained gels; i.e., probe hybridization or incorporation of labeled, or other detectable nucleotides.

One method of solving the problem of different optimal reaction conditions for the two steps involves the use of a high melting temperature wax (72°C) in the reaction tube to separate reagents as described in PCT Patent Publication No. WO 91/12342. The high melting temperature wax essentially enables the two individual reactions to occur at their own optimal conditions by separating a solution of EGTA and MgCl₂ from the RT reaction with a wax layer. The RT step is carried out at a temperature below the melting point of the wax, hence, the wax layer remains intact and the RT step is carried out using manganese at an optimal concentration. Upon completion of the RT step, the wax is melted during the first high temperature denaturation step of the PCR, thus releasing the EGTA to preferentially chelate the manganese and allow for a magnesium based DNA amplification. A modification of this technique employs microencapsulating the EGTA and magnesium into a bead made of the same high melting temperature wax.

In accordance with the present invention a metal buffer whose buffering agent acts as a chelating agent is used. Such a buffer binds manganese and thus allows a higher Mn²⁺ concentration to be used. The metal buffer may maintain the available Mn²⁺ concentration at a constant level over a wide range of added Mn²⁺. Metal buffering agents which can be useful in the methods of the present invention include N,N-bis(2-hydroxyethyl)glycine (bicine), N[tris(hydroxymethyl)methyl]glycine (tricine), acetate, glutamate, aspartate, N-hydroxyethyliminodiacetic acid (HIMDA), citrate, and isocitrate. A combination of buffering agents which provides similar metal binding and pH buffering capabilities as one of the exemplified buffers may also be suitable. The term "buffering agent," as used herein, is meant to encompass such combinations of buffering agents. In some cases, similar buffering effects may be achieved by altering the concentration of the buffering agent. For example, Example 13 describes the use of an elevated Tris concentration to achieve a broadening of the manganese range.

The metal buffering agent, which acts as a chelating agent, forms complexes with the metal cation in a reversible reaction. The stability of the chelator-metal complex, which represents the affinity of the chelator for the metal cation, is expressed as a "metal-buffer binding constant" or "stability constant", K_{M}. Because of the wide range of stability constants, it is more common to refer to the logarithm (base 10, written herein as Log) of the K_{M} of a metal buffer. The stability constant, K_{M}, is described in Good et al., 1966, Biochemistry 5:467-477, and Perrin and Dempsey, 1974, Chapter 7 in "Buffers for pH and Metal Ion Control", Chapman and Hall, New York, NY.

Suitable metal buffers that bind manganese for use in the methods of the present invention have a Log K_{M} (20°C, 0.1 M ionic strength) between 1 and 6 (i.e., 10 < K_{M} < 10⁶). Preferably the Log K_{M} is between 2 and 4, and most preferably between 2.5 and 3.5. A compilation of stability constants for a variety of chelating agents is provided in Martell and Smith, 1974, "Critical Stability Constants", Plenum Press, New York, NY, Vol 1; Martell and Smith, 1977, "Critical Stability Constants", Vol 3, Plenum Press, New York, pages 160-162; and Sillen and Martell, 1964, "Stability Constants of Metal-Ions Complexes", Spec. Publ. Chem. Soc. n17, page 458, London,

Preferred metal buffers are also capable of serving as hydrogen ion buffers in the pH range required in the methods of the present invention. Good et al., supra, described a class of buffers that serve both as metal cation and hydrogen ion buffers. The acid dissociation constant of a buffer, Kₐ, is described in Good et al., 1966, supra, and Perrin and Dempsey, 1974, supra. It is preferred that the buffer have a pKₐ, defined as -Log Kₐ, between 7 and 9 at 20°C and 0.1 M ionic strength, preferably between 7.5 and 8.5.

Preferred buffering agents for use in the methods of the present invention include N,N-bis(2-hydroxyethyl)glycine (bicine) and N[tris(hydroxymethyl)methyl]glycine (tricine). Both of these are zwitterionic aliphatic amines; more specifically, substituted glycines with carboxyl groups providing the Mn²⁺ binding ability. A comparison of properties of bicine, tricine, and Tris(hydroxymethyl)aminomethane (Tris) are shown below and found in Good et al., 1966, supra, which additionally provides the structures. All values are at 20°C and 0.1 M ionic strength.

| Buffer | pKₐ | delta-pKₐ/°C | Log K_{M} (Mn²⁺) |
|---|---|---|---|
| Tris | 8.3 | -0.031 | negligible |
| Bicine | 8.35 | -0.018 | 3.1 |
| Tricine | 8.15 | -0.021 | 2.7 |

The changes in the acid binding constant with temperature (delta-pKₐ) for bicine and tricine are significantly lower than for Tris-HCl. Tricine and Bicine also reduce the large temperature dependence of pH found with Tris.

Tricine and bicine buffers also broaden the usable Mn²⁺ concentration range in DNA polymerase incorporation assays. As the concentration of tricine or bicine is increased, the decrease in DNA polymerase activity at increasing Mn²⁺ concentration is less pronounced and the Mn²⁺ concentration optimum is shifted to a higher concentration. As shown in Example 7, using Tth DNA polymerase in a 50 mM bicine-KOH buffer (pH 8.3), manganese concentrations of 0.4 mM to 2.6 mM gave good incorporation on a DNA template. Using an RNA template, approximately 40% of maximum activity was observed with a Mn²⁺ concentration as low as 1 mM. Activity was observed to increase as the manganese concentration was increased from 1.0 to 6 mM and reached a plateau for concentrations between approximately 6 and 12.5 mM. A gradual decrease of activity was observed at higher Mn²⁺ concentrations; 48% of maximum activity was observed at 20 mM.

In the combined RT/PCR, a low free manganese ion concentration is needed for the DNA amplification step, but a high free manganese ion concentration is needed for the reverse transcription step. This dual effect of the tricine and bicine buffers of the present invention, that of extending the total allowed manganese concentration in a DNA amplification by complexing most of the free manganese ion, yet providing sufficient free manganese for the RT reaction, provides significant improvements to the RT/PCR. This is a very surprising result because expansion of the dual ranges would not have been predicted given the general theory and literature behind metal buffers.

Since reaction components such as dNTPs, primers, nucleic acids, proteins, EDTA, and many materials carried over from sample preparation procedures have the ability to chelate manganese, the control of a precise concentration of manganese is very difficult. Although these components can be controlled by the individual researcher (with great care), severe constraints are placed on manufacturing these reagents for diagnostic and research applications on a large scale. The use of the metal buffers tricine and bicine not only shifts the optimal Mn²⁺ concentration upwards, but also broadens the usable range of Mn²⁺ concentration and dNTP concentration. The ability to use a higher manganese concentration and a broader range of concentrations eases the problems of reagent manufacture and of precisely controlling the manganese concentration in a reaction.

Although the metal binding constant (Log K_{M}) of Tris is thought to be negligible (Good et al., 1966, supra), increasing the concentration of Tris-HCl in an RT/PCR from 10 mM to 100 mM can expand the Mn²⁺ concentration range on RNA targets. Although Tris buffer was thought to bind Mn²⁺ (as well as most other metals) negligibly, Morrison, 1979, Methods Enzymol. 24b:53-68, indicates that "the dissociation constant for the Mn-Tris complex is high at 250 mM, but in a solution of 100 mM Tris and 2 mM Mn²⁺ almost 29% of the metal ion would be chelated." Example 13 describes the use of 100 mM Tris in an RT/PCR which provided a significant broadening of the manganese concentration range wherein amplified product was observed. The amount of broadening was surprising and would not have been predicted given the general theory and literature behind metal buffers and Tris, in particular.

Bicine and tricine buffers preferably contain either KCl or KOAc as the monovalent cation. The bicine/KOAc buffer has a slightly lower ionic strength than the bicine/KCl buffer which could help destabilize secondary structures in a template with a high G+C content. The pH of the KOAc added to the reaction is not too critical because the bicine, which acts both as a metal buffer and a pH buffer, buffers the pH of the reaction adequately. Product was observed using KOAc between pH 7.0 and 9.4, with the optimum being pH 7.5. The final pH of a solution of 50 mM bicine (pH 8.3), 100 mM KOAc (pH 7.5), and 2.5 mM Mn(OAc)₂ is 7.97.

The bicine buffer solutions can be stored for extended lengths of time. For example, the product formed in an RT/PCR using a buffer diluted from a 10X solution of 500 mM bicine, 800 mM KCl, and 21 mM MgCl₂ stored at 4°C for 47 days was equal to the product formed in an RT/PCR using a freshly made buffer. The bicine maintains solubility of metal ions; an additional advantage of using the preferred bicine/KOAc/Mn(OAc)₂ buffer for RT/PCR is that Mn(OAc)₂ will have fewer solubility problems as compared to MnCl₂. Therefore, precipitation of manganese hydroxides and oxides, which are detrimental in RT/PCR, can be prevented.

The sterilization methods of the present invention specify that the RT/PCR be performed in the presence of dUTP. Maximum efficiency of uracil N-glycosylase (UNG) sterilization is achieved when dUMP is incorporated in place of every dTMP in the contaminating template. To maximize dUMP incorporation, it is desirable to minimize the amount of dTTP added to the RT/PCR because Tth DNA polymerase discriminates against dUTP when dTTP is present during reverse transcription by approximately 2-fold. Because dNTP binds Mn²⁺, the free Mn²⁺ concentration is directly related to the dNTP concentration. The bicine/KOAc buffer that increases the usable Mn²⁺ concentration range also allows an increased concentration of dNTP to be used at a given Mn²⁺ concentration because the buffering of the free Mn²⁺ concentration compensates for the additional Mn²⁺ binding by the dNTP. The bicine/KOAc buffer not only allows increased total dNTP concentrations, but also allows higher relative concentrations of dUTP to be used during the RT/PCR to increase the levels of incorporation of dUMP during the reverse transcription step by rTth DNA polymerase. This is particularly advantageous in RNA targets that have a large percentage of adenine residues, such as human immunodeficiency virus (HIV).

A further advantage of the preferred bicine and trichinae buffers of the present invention is that they increase the efficiency of the sterilization methods. Both divalent metal cations and high ionic strength are known to inhibit UNG (Lindahl et al., 1977, J. BioL Chem. 252(10):3286-3294, Krokan and Witter, 1981, NucL Acids Res. 9(11):2599-2613, and Caradonna and Cheng, 1980, J. Biol. Chem. 255(6):2293-2300).

The preferred buffers reduce both the free metal cation level and the total ionic strength, thereby minimizing the inhibitory effect of the buffer on UNG and improving the efficiency of the sterilization of the reaction mixture.

Metal ion catalyzed hydrolysis of the RNA template decreases the efficiency of the RT reaction and limits the length of template that can be reverse transcribed. The problem of template hydrolysis is aggravated by the high temperature of the RT step in the present methods. Methods and reaction conditions using MnCl₂ in a Tris buffer which minimize the hydrolysis of RNA templates are provided in the Examples and discussed in Myers and Gelfand, 1991, Biochemistry 30:7661-7666.
The preferred bicine/KOAc buffers complex the manganese such that less metal catalyzed RNA hydrolysis occurs at elevated temperatures. Furthermore, little if any additional loss of full-length labeled RNA occurs by including a 15 second, 95°C preincubation of the RNA when using these buffers. The preferred buffers allow increasing the reverse transcription reaction time to increase efficiency in the RT step and including a high temperature preincubation to denature the RNA to relieve high degrees of secondary structure prior to reverse transcription or, in the case that double-stranded RNA template is to be amplified, to denature the template to provide a single-stranded template for the reverse transcription. Furthermore, decreasing RNA hydrolysis facilitates the synthesis of long (>2 kb) cDNA using Tth DNA polymerase by decreasing the chance that the RNA template is degraded before reverse transcription is completed.

The present methods require only that RNA is present in the sample. In an example, a synthetic RNA prepared using a plasmid containing a bacteriophage T7 promoter is reverse transcribed and amplified by the methods of the present invention. In another example, a heterogeneous population of total cellular RNA is used to reverse transcribe and amplify a specific mRNA. For practicing the invention the amount of RNA present in the sample is generally within the range of 0.1 pg to 1 µg. The amount required and the results will vary depending on the complexity of the sample RNA and the type of detection utilized. Because of the specificity of the high temperature reverse transcription reaction, 1 to 10⁸ molecules of the target RNA are sufficient to provide up to or exceeding microgram quantities of PCR product. In several of the disclosed examples, amplification products are visualized by ethidium bromide staining after gel electrophoresis of 5% of the total reaction mix. Thus, the amount of amplified target required is substantially reduced when alternative means for assay of the product are utilized. For example, isotopically labeled DNA probes suitable for detecting the electrophoresed PCR products would increase the sensitivity of detection and therefore decrease the amount of amplification or number of cycles required to detect the amplified product (e.g., 1-10⁸ molecules of target RNA in the sample).

Preferably, the amount of RNA present in a 20 µl 10 mM Tris-HCl reverse transcription reaction is 10 pg to 500 ng and most preferably 0.1 to 300 ng. When the sample contains more than 300 ng of RNA, it may be desirable to include additional enzyme to ensure transcription of a full length cDNA product from the RNA template. However, if the reverse transcription reaction is coupled to PCR, the effect of high enzyme concentration in the PCR reaction should be considered. For example when Taq DNA polymerase is used as the thermoactive polymerase, high enzyme concentrations can result in non-specific PCR products and reduced yields (see Saiki in "PCR Technology" Ed. Erlich, 1989, Stockton Press). The potential problems resulting from a high enzyme concentration, however, are easily avoided by inactivating the thermoactive DNA polymerase between the reverse transcription reaction and the amplification reaction. Inactivation is achieved by incubating the cDNA synthesis reaction mix at 99°C for 3 to 10 minutes. An appropriate amount of thermostable DNA polymerase is then added back to the reaction mix, and PCR is conducted as usuaL This method is also suitable when different thermostable DNA polymerases are used for each of the two reactions (see Example VII in WO 91/09944).

An advantage of the bicine buffers described herein is that higher amounts of RNA may be present in the reverse transcription reaction. For example, the preferred amount of RNA using a bicine buffer and dTTP is up to 1 µg for a 50 µl reaction. In the preferred range, 1 to 10 units of thermoactive DNA polymerase is sufficient for providing a full length cDNA product. To achieve predominantly full length cDNA, the enzyme to template ratio is preferably greater than 0.5.

An advantage of the RT/PCR methods using Tth or Taq DNA polymerase is that lower molar concentrations of the DNA polymerase are needed for efficient reverse transcription and amplification. For example, each unit of activity requires about 1.0 pmole of E. coli DNA polymerase I, whereas only about 0.043 pmole of Taq DNA polymerase is required, or about 20- to 25-fold less protein. Example 3 describes a homogeneous RT/PCR which uses 5 units of rTth DNA polymerase in a 20 µl reaction, corresponding to approximately a 15 nM DNA polymerase concentration. Using the preferred bicine and tricine buffers, the amount of DNA polymerase can be reduced further. Examples 8, 10 and 12 describe homogeneous RT/PCR using 10 units rTth DNA polymerase in 100 µl reactions, corresponding to approximately a 6 nM DNA polymerase concentration. Furthermore, the one enzyme RT/PCR described herein requires considerably less total protein in the reaction compared to multi-enzyme amplification systems such as 3SR (Kwoh et al., supra., Guatelli et al., supra., and PCT Patent Publication No. WO 92/0880A). Whereas the exemplified 100 µl RT/PCR reactions contain 40 ng (10 units) of rTth DNA polymerase, a 100 µl 3SR reaction would contain 1.44 µg of enzyme (0.6 µg AMV-RT, 0.83 µg T7 RNA polymerase, and 0.01 µg E. coli RNase H) or about 36 times more protein. Both the decrease in total protein and the use of only one enzyme in the homogeneous RT/PCR significantly simplify problems of reagent manufacture and quality control.

Once the sample containing RNA has been prepared and the suitable primer and salts have been added, the reaction is incubated with the thermoactive DNA polymerase for 1-60 minutes. Usually, however, a reaction time of 2 to 30 minutes is suitable. If the target molecule is long, or if the ratio of total RNA to target RNA is high, e.g., 100 copies of target RNA in the presence of 250 ng of total RNA, an incubation time of 10-30 minutes is preferred.

It is preferred, but not essential that the thermoactive DNA polymerase is added to the reverse transcription reaction mix after both the primer and the RNA template are added. Alternatively, for example, the enzyme and primer are added last, or the MnCl₂, or template plus MnCl₂ are added last. It is generally desirable that at least one component that is essential for polymerization activity not be present, until such time as the primer and template are both present and the enzyme can bind to and extend the desired primer/template substrate (see European Patent Application EP-A-515,506).

In practicing the present methods the reaction mix is incubated above 40°C, although a preferred temperature is 55-75°C. At this temperature, the specificity of the primer-template annealing is enhanced over the annealing specificity at a lower temperature, and the thermoactive enzyme has higher activity at the elevated temperature. The elevated temperature reduces non-specific priming by degraded native nucleic acid and by incorrect primer-template hybridization.

Following reverse transcription, the RNA template may be degraded or alternatively denatured, providing a template for continuous replication resulting in an excess of single-stranded DNA molecules. This excess of single-stranded DNA can be detected by standard probe hybridization techniques. Thus, the invention provides means for direct detection of target segments. The resulting nucleic acid products can be detected by a number of electrophoretic or chromatographic means. The use of a radiolabeled triphosphate is helpful in monitoring the extent of the reaction and the size of products formed, although this is not an essential component of the invention.

The reverse transcription reaction products are suitable as templates for amplification by PCR. In one embodiment of the invention, following the high temperature reverse transcription incubation, the reverse transcription reaction is adjusted to PCR buffering conditions, and the amplification reaction is initiated following the addition of a second primer. PCR buffer is added to maintain the appropriate buffering capacity, pH, monovalent cation concentration, and to dilute the concentration of enzyme and dNTPs to within 20-200 µM each dNTP. MgCl₂ is added to a final concentration of 1-3 mM. Preferably, the concentrations of dNTPs in both the reverse transcriptase and PCR reaction mixes are balanced. Because Mn²⁺ can diminish PCR amplification when present at high concentrations, in a preferred embodiment of the invention the Mn²⁺ is chelated prior to the PCR amplification. The presence of high amounts of Mn²⁺ also may decrease fidelity during amplification, however chelating the Mn²⁺ avoids this problem. Accordingly, in a preferred embodiment, following the reverse transcription reaction, EGTA is added to a concentration between about 1-3 times the molar concentration of Mn²⁺ to chelate the Mn²⁺. In the presence of both Mg²⁺ and Mn²⁺, EGTA preferentially binds Mn²⁺. Low dNTP and Mg²⁺ concentrations, as described herein, may also increase fidelity of Tth DNA polymerase during amplification. Glycerol and non-ionic detergent (for example, Tween-20™) may be added to a final concentration of between 1-20% and 0.01-0.05%, respectively, to increase enzyme stability.

In an alternative preferred embodiment, Mn²⁺ is not chelated prior to PCR. PCR can utilize Mn²⁺ in place of Mg²⁺, although Mg²⁺ is preferred as described above. In particular, for applications such as, for example, large scale diagnostic screening, the risk of infidelity during amplification and potential low level hydrolysis of template are readily tolerated in view of the tremendous advantages a homogeneous RT/PCR method provides. The two-step single addition procedure minimizes sample handling and reduces the potential for cross contamination. Because MnCl₂ affects PCR efficiency, the optimum concentration is preferably titrated by standard means for the particular reaction components utilized: primers, target concentration, dNTP concentration, buffers, salts etc. In a preferred embodiment of the invention, a bicine/KOAc buffer containing Mn(OAc)₂ is used which allows for a wider range of Mn²⁺ and dNTP concentrations.

The methods provided herein have numerous applications, particularly in the fields of molecular biology and medical diagnostics. The reverse transcriptase activity described provides a cDNA transcript from an RNA template. The methods for production and amplification of DNA segments from an RNA molecule are suitable where the RNA molecule is a member of a population of total RNA or is present in a small amount in a biological sample. Detection of a specific RNA molecule present in a sample is greatly facilitated by a thermoactive or thermostable DNA polymerase used in the methods described herein. The enormous increase in specificity obviates the need for "nested PCR" to detect rare targets. A specific RNA molecule or a total population of RNA molecules can be amplified, quantitated, isolated, and, if desired, cloned and sequenced using a thermoactive or thermostable enzyme as described herein.

The methods and compositions of the present invention are a vast improvement over prior methods of reverse transcribing RNA into a DNA product When starting with an RNA template, these methods have enhanced specificity and provide templates for PCR amplification that are produced more efficiently than by previously available methods. The invention provides more specific and, therefore, more accurate means for detection and characterization of specific ribonucleic acid sequences, such as those associated with infectious diseases, genetic disorders, or cellular disorders.

Those skilled in the art will recognize that the compositions of the instant invention can be incorporated into kits. Thus, the invention relates also to kits comprising a buffer with a suitable buffering agent which is a zwitterionic compound providing hydrogen buffering and preferably buffer also the divalent cation concentration and a thermoactive DNA polymerase as well as preferably instructions describing the method for using the same for reverse transcribing RNA. In one embodiment such a kit may relate to the detection of at least one specific target RNA sequence in a sample. Such a kit would comprise, in addition to the elements listed above, a primer comprising a sequence sufficiently complimentary to a specific target RNA sequence to hybridize therewith. Diagnostic kits for the amplification and detection of at least one specific RNA sequence in a sample may comprise a primer having a sequence sufficiently identical with the RNA target to hybridize with the first strand of cDNA synthesized to prime synthesis of a second cDNA strand. Kits may contain, in addition to the components listed, the four deoxyribonucleotide triphosphates, suitable buffers as described herein, oligo(dT), RNase H, linkers for cloning, as well as one or more restriction enzymes.

The following examples are offered by way of illustration only and should not be construed as intending to limit the invention disclosed in the present patent application in any manner. Further details on the materials and methods used in these examples may be found in WO 91/09944. In particular WO 91/09944 provides detailed information on the synthetic template pAW106 and the primers DM156 (SEQ ID No. 9), DM151 (SEQ ID No. 10), DM152 (SEQ ID No. 11) and TM01 (SEQ ID No. 12), whereby the sequence of these primers is as follows:

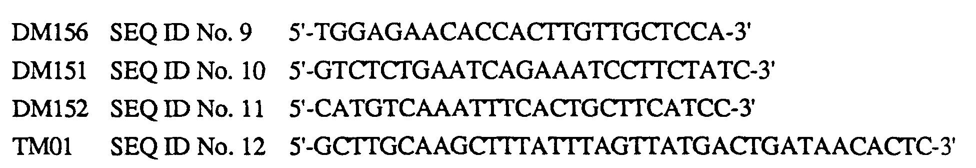

### Example 1

### Comparison of Taq Polymerase and Tth Polymerase in a Coupled RT/PCR Reaction

The use of the cRNA standard described in Example VII of WO 91/09944 facilitates direct analysis of the effect of experimental conditions on RT/PCR efficiency, because the number of target molecules present in the reaction mix is known. Specifically, the efficiency of Tth and Taq DNA polymerases in a coupled RT/PCR reaction were compared.

RT reactions (20 µl) contained 10 mM Tris-HCl, pH 8.3; 90 mM KCl (40 mM for reactions containing Taq); 1.0 mM MnCl₂; 200 µM each of dATP, dCTP, dGTP, and dTTP; 15 pmol of DM152 (SEQ ID No. 11) and 5 units of rTth or Taq DNA polymerase, and 10⁶, 10⁵, or 10⁴ copies of pAW109 cRNA. The six reactions were overlaid with 75 µl mineral oil and incubated for 15 minutes 70°C.

Following the RT reaction, 80 µl of a solution containing 10 mM Tris-HCl (pH 8.3), 100 mM KCl (50 mM for reactions containing Taq), 1.88 mM MgCl₂, 0.75 mM EGTA, 5% glycerol [v/v], and 15 pmol of primer DM151 was added. The samples (100 µl) were then amplified in a Perkin Elmer, Norwalk, CT, Thermal Cycler as follows: 2 minutes at 95°C for 1 cycle; 1 minute at 95°C and 1 minute at 60°C for 35 cycles; and 7 minutes at 60°C for 1 cycle. Aliquots (5 µl) of the PCR amplifications were analyzed by electrophoresis on 2% (w/v) NuSieve® 1% (w/v) Seakem® agarose stained with ethidium bromide.

### Results

The rTth DNA polymerase generated a 308 bp product visualized by ethidium bromide stained gel electrophoresis starting with 10⁴ copies of target cRNA. Product was not observed for the Taq polymerase at 10⁴ or 10⁵ copies of target, although lower limits of detection would be expected if hybridization techniques were used rather than ethidium bromide staining. These results demonstrated that under similar reaction conditions the Tth DNA polymerase provides approximately 100-fold greater sensitivity than the analogous Taq DNA polymerase in a coupled reverse transcription PCR amplification.

### Example 2

### Preferred Non-Homogeneous Reverse Transcription/PCR Protocol

### A. Reverse Transcription Reaction

In a 0.5 ml polypropylene microcentrifuge tube combine 9.4 µl sterile distilled water; 2 µl 10X rTth RT buffer; 2 µl MnCl₂ (10 mM); 0.4 µl of each of dGTP, dATP, dTTP, and dCTP (each at 10 mM); 2 µl rTth DNA polymerase 2.5 U/µl; 1 µl of primer DM152 (SEQ ID No. 11) (15 µM) (or an alternative "downstream" primer); and 2 µl positive control RNA or experimental sample containing ≤ 250 ng total RNA.

In this embodiment, the positive control RNA serves as template for DM152 (SEQ ID No. 11). The control RNA concentration is preferably ∼10⁴ copies/20 µl. For example, the control RNA may be RNA transcribed from pAW109 in 30 µg/ml E. coli rRNA in 10 mM Tris-HCl, pH 8.0, 1 mM EDTA and 10 mM NaCl.

The total reverse transcription reaction volume should be 20 µl per sample.

To reduce evaporation or refluxing, overlay the mix with 50-100 µl mineral oil.

Incubate the tubes in a Perkin-Elmer, Norwalk, CT, thermal cycler using a soak file at 70°C for 5-15 minutes. Stop the reaction by placing the tubes on ice until needed.

### B. PCR Reaction

For each sample prepare a minimum of 80 µl of PCR master mix as follows: 8 µl 10X chelating buffer, 6-10 µl 25 mM MgCl₂, 1 µl primer DM151 (SEQ ID No. 10) (15 µM) or experimental "upstream" primer and sterile distilled water. Any combination of water, MgCl₂ and "upstream" primer volumes can be used as long as the total volume of the master mix equals 80 µl per sample.

The optimal MgCl₂ concentration may vary, depending on the total dNTP concentration, and the primer and template used. In most cases a final concentration of MgCl₂ in the range of 1.5-2.5 mM in the reaction mix will provide excellent results. If the template used is the positive control pAW109 RNA, 6 µl of 25 mM MgCl₂ stock solution is preferred for final 1.5 mM MgCl₂ concentration.

Dispense 80 µl of the PCR master mix into each reverse transcription reaction tube. Change pipet tips between additions to avoid sample carryover. Centrifuge the tubes for ∼30 seconds in a microcentrifuge.

For amplification of the pAW109 RNA positive control, the thermal cycler (Perkin Elmer, Norwalk, CT) is programmed for four linked files as follows:

| | |
|---|---|
| Step Cycle | 2 minutes at 95°C for 1 cycle |
| Step Cycle | 1 minute at 95°C and 1 minute at 60°C for 35 cycles |
| Step Cycle | 7 minutes at 60°C for 1 cycle |
| Soak | 4°C |

The PCR amplified samples can be stored frozen until subsequent analysis.

The selection of 60°C for the anneal-extend temperature is optimal for amplification of the positive control cDNA. It may be. necessary to lower or raise the anneal-extend temperature for other primer-template pairs. Higher anneal-extend temperatures generally result in improved product specificity (see Saiki et al., 1988, Science 239:487-491). The optimum can be determined empirically by testing at 5°C, or smaller, increments until the maximum in specificity and product yield is reached.

The optimal magnesium chloride concentration for PCR amplification can be determined empirically by testing concentrations from 1.5 to 2.5 mM magnesium chloride for each primer set. Too little or too much magnesium chloride may effect amplification efficiency. It may be preferable to adjust the magnesium chloride concentration in parallel with substantial changes in the concentration of sample RNA, dNTPs, cDNA, and DNA.

For templates known to contain a high amount of secondary structure, a "hot start" protocol may be preferred. Two reaction mixes for the reverse transcription reaction are prepared. Mix A: 9.4 µl sterile distilled water, 2 µl 10X rTth reverse transcriptase buffer, 1 µl "downstream primer;" 2 µl sample RNA (<250 ng of total RNA). Mix B: 2 µl, 10 mM MnCl₂ solution; 0.4 µl dGTP; 0.4 µl dATP; 0.4 µl dCTP: 0.4 µl dTTP (each at 10 mM); 2 µl rTth DNA polymerase.

Prepare both reaction mixes at room temperature. Incubate Mix A for 5 minutes at 70°C, add reaction Mix B (while reaction Mix A is still at 70°C) and incubate for 5 to 15 minutes at 70°C as described above in the section entitled "Reverse Transcription Reaction." Run the PCR reaction as described above.

### C. Reagents

The preferred protocol utilizes the following reagents:

| | |
|---|---|
| rTth DNA polymerase | 2.5 Units/µl |
| Primer DM152 (SEQ ID No. 11) | 15 µM |
| Primer DM151 (SEQ ID No. 10) | 15 µM |
| Positive Control RNA | 5 x 10³ copies/µl |
| dATP | 10 mM |
| dGTP | 10 mM |
| dCTP | 10 mM |
| dTTP | 10 mM |
| 10X rTth Reverse | 100 mM Tris-HCl |
| Transcriptase RT Buffer: | pH 8.3, 900 mM KCl |
| 10X Chelating Buffer: | 50% glycerol (v/v) |
| | 100 mM Tris-HCl, pH 8.3, 1 M KCl, |
| | 7.5 mM EGTA, 0.5% Tween 20 |
| MnCl₂ Solution | 10 mM |
| MgCl₂ Solution | 25 mM |

These components may be assembled as a kit for high temperature reverse transcription. Variations to the kit are within the scope of the disclosed invention. For example, MnCl₂ may be included in the reverse transcriptase buffer and MgCl₂ may be included in the Chelating buffer. However, for optimization of the reactions MnCl₂ and MgCl₂ are provided as separate reagents. The use of a positive control, while not essential, is preferred in a commercial embodiment of the invention.

### Example 3

### Homogeneous RT/PCR Assay

This method provides a procedure for a two-step, single addition reverse transcription/PCR amplification reaction. A TC9600 thermal cycler (Perkin Elmer, Norwalk, CT) was used and the instrument was turned on, to preheat the cover, prior to preparing the reaction mixture. Reactions were carried out in 0.2 ml MicroAmp® tubes, commercially available from Perkin Elmer, Norwalk, CT. Each reaction contained 6.4 µl sterile distilled H₂O; 2 µl 10X RT buffer (100 mM Tris-HCl, pH 8.3; 900 mM KCl); 1.6 µl of 10 mM MnCl₂; 2 µl of 10X dNTP-T (2 mM each dATP, dCTP, dGTP in H₂O pH 7.0); 2 µl of 2 mM dTTP; 1 µl of primer DM152 (SEQ ID No. 11) (15 µM); 1 µl of primer DM151 (15 µM); and 2 µl rTth DNA polymerase (2.5 U/µl). A 20X reaction mixture was made up (360 µl total volume) and 18 µl of the mixture was aliquoted into 16 tubes containing template as described below. The template used was AW109 cRNA. Tube Nos. 1-3 and 9-11 contained 10⁴ copies of template in 2 µl. Tube Nos. 4-6 and 12-14 each contained 10² copies in 2 µl. Tube Nos. 7, 8, 15, and 16 contained only 2 µl of 30 ng/µl rRNA as a negative control.

Tube Nos. 1-8 were kept on ice during the RT reaction as -RT controls. Tube Nos. 9-16 were placed in a TC9600 thermal cycler (Perkin Elmer, Norwalk, CT) and heated for 1 cycle at 70°C for 15 minutes and then heated to 95°C while Tube Nos. 1-8 were placed in the thermal cycler for the PCR step. All tubes were cycled as follows:
75 seconds 95°C 1 cycle
30 seconds 95°C, 20 seconds 60°C for 35 cycles
2 minutes 60°C 1 cycle

### Results

Five µl of each reaction was then analyzed on a 2% NuSieve 1% agarose gel, stained with ethidium bromide and photographed. No product of the predicted size was visible in the -RT controls (Tube Nos. 1-8) or the "no target controls" (Tube Nos. 15 and 16). Product of the expected size was readily visible in lanes 9-11 (10⁴ copies of target) and also present in lanes 12-14 (10² copies of target), although, expectedly, with less intensity.

### Example 4

### Utilization of dUTP and Uracil-N-Glycosylase (UNG) as a PCR Carryover Prevention During High Temperature Reverse Transcription and Amplification

This example illustrates the incorporation of an unconventional nucleotide to minimize carryover contamination. The reaction mix was treated with UNG prior to reverse transcription to degrade contaminating products from previous assays containing the same unconventional nucleotide. UNG treatment is as follows: 0.5 units UNG (developed and manufactured by Hoffmann-La Roche Inc. and commercially available from Perkin Elmer, Norwalk, CT) per 20 µl RT reaction. The reaction was incubated for 10 minutes at room temperature followed by heating at 70°C for 15 minutes to inactivate the glycosylase and allow for reverse transcription. The experiment also demonstrates MnCl₂ concentration titration for determining the optimum concentration for the particular target, primers, and reaction conditions shown. The cDNA is then amplified by a PCR.

An 8X RT reaction mixture was prepared that contained: 48 µl sterile DEPC-treated distilled water, 16 µl 10X RT Buffer (100 mM Tris-HCl, pH 8.3; 900 mM KCl); 16 µl of a dNTP mix containing 2 mM each of dATP, dCTP, dGTP, and dUTP; 16 µl each of DM152 (SEQ ID No. 11) (1.5 µM) and DM151 (SEQ ID No. 10) (1.5 µM); 16 µl of AW109 cRNA template (5 x 10³ copies/µl); and 16 µl of rTth DNA polymerase (2.5 units/µl). The final volume was 144 µl (18 µl/reaction). A 7X PCR master mixture was prepared that contained: 297 µl sterile DEPC-treated distilled water, 56 µl 10X PCR buffer (100 mM Tris-HCl, pH 8.3; 1 M KCl; 7.5 mM EGTA; 50% glycerol [v/v]); 140 µl 10 mM MgCl₂; 56 µl dNTP mix containing 2 mM each of dATP, dCTP, dGTP, dUTP; 5.6 µl of each of DM152 and DM151 (SEQ ID Nos. 11 and 10) (15 µM). The final volume was 560 µl, 80 µl per reaction.

Eighteen µl of the RT mix was aliquoted into six sterile microcentrifuge tubes and MnCl₂ added in a 2 µl volume to provide a final MnCl₂ concentration as follows: Tube Nos. 1 and 2 (1.2 mM MnCl₂); Tube Nos. 3 and 4 (1.0 mM MnCl₂); and Tube Nos. 5 and 6 (0.8 mM MnCl₂). A mineral oil overlay (75 µl) was added to each tube and the reactions were incubated at 70°C for 15 minutes in a water bath. Following the 70°C incubation, 80 µl of the PCR master mix was added to each. The reaction tubes were thermocycled as follows: 2 minutes at 95°C for 1 cycle; 1 minute at 95°C and 1 minute at 60°C for 35 cycles; 7 minutes at 60°C for 1 cycle; and soak at 4°C.

### Results

Five µl of each reaction mix was electrophoresed on a 2% NuSieve 1% agarose gel. The gel was stained and photographed. PCR product of the expected size was clearly visible in samples from all three MnCl₂ concentrations. The product yield increased with increasing MnCl₂ concentration.

### Example 5

### Procedure for Sterilization of a Homogeneous RT/PCR Assay

This example illustrates a method for sterilization of a homogeneous RT/PCR reaction contaminated with nucleic acids generated from a previous reaction. The reaction mix is treated with UNG prior to reverse transcription.

The unconventional nucleotide, dUTP, is incorporated during the RT/PCR reaction. Consequently, any product DNA present as a contaminant in subsequent reactions can be hydrolyzed using UNG.

In a 0.2 ml MicroAmp® tube combine 5.5 µl sterile distilled water, 2 µl 10X RT buffer (100 mM Tris-HCl, pH 8.3; 900 mM KCl); 2 µl of 8 mM MnCl₂; 2 µl dNTP mix containing 2 mM each of dATP, dCTP, dGTP, and dUTP; 2 µl each of DM152 (SEQ ID No. 11) (1.5 µM) and DM151 (SEQ ID No. 10) (1.5 µM); 2 µl of AW109 cRNA template (5 x 10³ copies/µl); 0.5 µl UNG (1 unit/µl); and 2 µl of rTth (2.5 units/µl). The reaction is incubated for 10 minutes at room temperature and subsequently heated at 70°C for 15 minutes to inactivate the glycosylase prior to reverse transcription. The cDNA is then amplified by a PCR.

In this example, the positive control RNA serves as a template for DM152 (SEQ ID No. 11) and DM151 (SEQ ID No. 10) is the upstream primer. The total reaction volume is 20 µl/sample. Incubate the tubes in a Thermal Cycler (for example, a TC9600 thermal cycler [Perkin Elmer, Norwalk, CT]) as follows:
70°C for 15 minutes for 1 cycle
95°C for 15 seconds and 60° for 20 seconds for 2 cycles
90°C for 15 seconds and 60°C for 20 seconds for 33 cycles
60°C for 4 minutes for 1 cycle
The optimal manganese concentration may vary depending on the particular sample, target, primers, and the dNTP concentration in the reaction mixture.

### Example 6

### Additional Template Nucleic Acids

Additional RNA and DNA templates were used in the experiments described in the Examples, below.

### I. pTM3

The plasmid pTM3 is a 7821 nucleotide circular single stranded DNA with approximately 700 nucleotides of pAW109 DNA present. This provides a DNA template with the same sequence and primer binding site as the pAW109 cRNA. When transcribed using primer MT24 (SEQ ID No. 13), the first 253 nucleotides are identical to pAW109 cRNA. Beyond that region, the DNA becomes G+C rich and comprises DNA from Thermus aquaticus, including the gene for the Taq DNA polymerase. The pTM3 plasmid was constructed using the following protocol using techniques well known in the art (see Sambrook (see Sambrook et al., Molecular Cloning - A Laboratory Manual. Cold Spring Harbor Laboratory, Cold Spring Harbor, New York, 1989).

The insert was prepared by linearizing pAW109 DNA, described above, with BamHI. Linker adapters MT20 (SEQ ID No. 14) and MT21 (SEQ ID No. 15) were annealed to the linearized pAW109 DNA and ligated. These linkers anneal to the BamHI site. The fragment was digested with EcoRI and the resulting 706 bp fragment was gel purified.

The expression vector pLSG1 is described in U.S. Patent No. 4,889,818, and contains the gene encoding the Taq DNA polymerase. The plasmid pLSG1 was linearized with EcoRI, mixed with an excess amount of gel purified fragment, and ligated to the fragment. The resulting plasmid was transformed into DG98 and single-stranded DNA was isolated with a helper phage (described in U.S. Patent Nos. 4,889,818 and 5,079,352 and Lawyer et al., 1993, supra.).

The oligonucleotide sequences used in the above reactions are provided below.

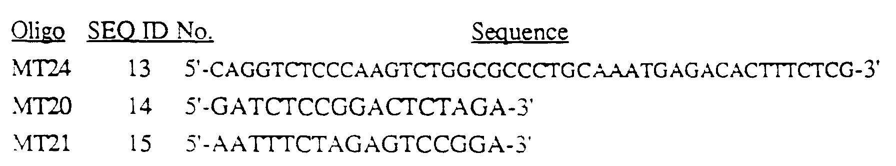

### II. HCV

HCV RNA transcript was generated as described in Young et al., 1993, J. Clin. Microbiol. 31:882-886. The cDNA clone was designated therein as pHCV1.1A. Preferred primers for the amplification of HCV templates are KY78 (SEQ ID No. 16; 5'-CTCGCAAGCACCCTATCAGGCAGT-3') and KY90 (SEQ ID No. 17; 5'-GCAGAAAGCGTCTAGCCATGGCGT-3'). KY78 (SEQ ID No. 16) and KY90 (SEQ ID No. 17) are biotinylated at the 5' end: KY80 (SEQ ID No. 17) is a nonbiotinylated version of KY90 (SEQ ID No. 17).

### III. HIV

A template was designed with primer binding regions identical to HIV-1 and an internal region flanked by the primer binding sites with a nucleotide sequence that, while maintaining the same base composition, differed from the corresponding HIV-1 sequence sufficiently to allow detection by a unique sequence specific probe. Preferred primers for the amplification of HIV templates are the 5'-biotinylated derivatives of SK431 (SEQ ID No. 6) and SK462 (SEQ ID No. 5), described above.

The template was generated by the annealing and extension of two oligonucleotides that overlap by 8 bases of complementarity at the 3' termini. The constituent oligonucleotides can be synthesized by any of the means for synthesizing oligonucleotides described above. The first oligonucleotide, SK550 (SEQ ID No. 18), contains a Sail linker and a SK462 (SEQ ID No. 5) primer binding region. The second oligonucleotide, SK551 (SEQ ID No. 19), contains a SK431 (SEQ ID No. 6) primer binding region. Synthesis of the control template was carried out using techniques well known in the art (see Sambrook et al., 1989, supra.).

The reaction mixture for the annealing and extension reaction was as follows: 7 µl of 10X Polymerase buffer (100 mM Tris-HCl at pH 7.5, 500 mM sodium chloride (NaCl), 100 mM magnesium acetate [Mg(OAc)2]).
50 pmoles SK550 (SEQ ID No. 18)
50 pmoles SK551 (SEQ ID No. 19)
15 µl of each dATP, dGTP, dCTP, dTTP (10 mM stock solutions)
1 µl Klenow Fragment (5 U)
H20 to 70 µl

The two oligonucleotides were mixed and held on ice for 10 minutes to allow the 3' termini of each oligonucleotide to anneal. The extension reaction was carried out for 30 minutes at room temperature followed by 30 minutes at 37°C. Following extension, the reaction mixture was held at 72°C for 10 minutes to inactivate the polymerase.

The extended products were digested with Sail, which cleaves the SK550 (SEQ ID No. 18) end of the duplex sequence; the SK551 (SEQ ID No. 19) end remains blunt. The resulting fragment was cloned into the SalI and SmaI sites of the transcription vector pSP64 (Promega, Madison, WI) (with poly A), resulting in plasmid pNAS-2.

Following isolation and purification, pNAS-2 was linearized by digestion with EcoRI and transcribed in vitro with SP6 RNA polymerase. To remove residual DNA, the RNA was digested with RNase-free DNase and passed through an oligo-dT column.

The nucleotide sequences used in the above reactions are provided below.

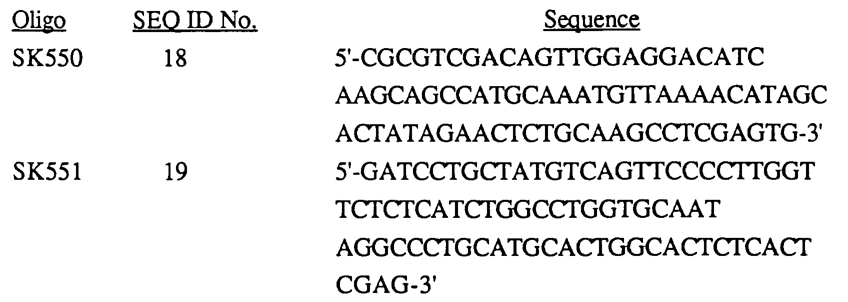

### Example 7

### Manganese Concentration Range

Extension reactions using RNA and DNA templates in either a Bicine or a Tris buffer were performed to determine the usable range of Mn²⁺ concentrations for each reaction. A series of Mn²⁺ concentrations for extension reactions with a DNA template and extension reactions with an RNA template were used. All reactions were carried out at 60°C for 10 minutes in a 20 µl total volume. Reaction conditions were as follows:

### RNA template, Bicine Buffer

3 x 10¹¹ copies pAW109 cRNA
0.125 µM MT24 (SEQ ID No. 13)
300 µM each dATP, dCTP, dGTP, dTTP
50 mM Bicine-KOH (pH 8.3)
100 mM KOAc (pH 7.5)
Mn(OAc)₂ (1-20 mM, 1-6 mM shown)
5 U rTth* DNA polymerase

### RNA template, Tris Buffer

3 x 10¹¹ copies pAW109 cRNA
0.125 µM MT24 (SEQ ID No. 13)
200 µM each dATP, dCTP, dGTP, dTTP
10 mM Tris-HCl (pH 8.3)
90 mM KCl
MnCl₂ (0.4-2.5 mM)
5 U rTth* DNA polymerase

### DNA template. Bicine Buffer

1.5 x 10¹¹ copies pTM3 ss DNA
0.0625 µM MT24 (SEQ ID No. 13)
300 µM each dATP, dCTP, dGTP, dTTP
50 mM Bicine-KOH (pH 8.3)
100 mM KOAc (pH 7.5)
Mn(OAc)₂ (1-5 mM)
0.15 U rTth* DNA polymerase

### DNA template. Tris Buffer

1.5 x 10¹¹ copies pTM3 ss DNA
0.0625 µM MT24 (SEQ ID No. 13)
200 µM each dATP, dCTP, dGTP. dTTP
10 mM Tris-HCl (pH 8.3)
90 mM KCl
MnCl₂ (0.4-2.5 mM)
0.15 U rTth* DNA polymerase

* Developed and manufactured by Hoffmann-La Roche Inc. and commercially available from Perkin Elmer, Norwalk, CT.

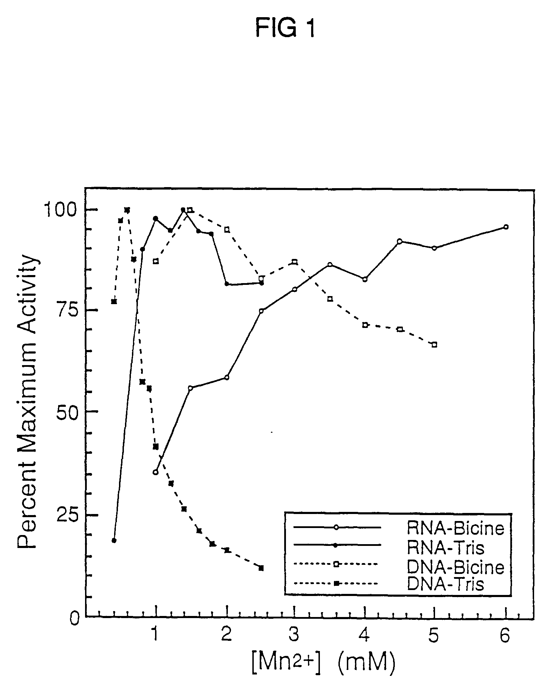

The amount of dNMP incorporated was assayed as described in Myers and Gelfand, 1991, supra, The results are shown in Figure 1. The amount of dNMP incorporated is presented as a percentage of the maximum amount incorporated for each reaction. The maximum amount incorporated for each reactions is given below.

| Template | Buffer | 100% Activity |
|---|---|---|
| RNA | Bicine | 98 pmol dNMP incorporated |
| RNA | Tris | 87 pmol dNMP incorporated |
| DNA | Bicine | 166 pmol dNMP incorporated |
| DNA | Tris | 173 pmol dNMP incorporated |

Using the Tris buffer, the manganese concentration providing optimal synthesis with DNA templates was found to be approximately 0.6 mM, the enzyme obtained maximal reverse transcriptase activity with the RNA template at approximately 1.4 mM manganese. Substituting the bicine buffer both increased and broadened the optimal Mn²⁺ concentration for each reaction. Using the bicine buffer, maximum synthesis with DNA templates was shifted to 1.5 mM manganese, while increases in the amount synthesized with RNA templates were seen up to 6 mM manganese. Although the Mn²⁺ optima for rTth DNA polymerase reverse transcriptase activity on RNA templates and DNA polymerase activity on DNA templates are still different for the individual reactions when using a bicine buffer, a single Mn²⁺ concentration of about 3.2 mM for a homogeneous RT/PCR appears to be at least as efficient as the RT/PCR conditions using a Tris buffer described in Example 3, above. However, the range of usable manganese concentrations for each reaction has been greatly expanded. This is a surprising result because expansion of the dual range would not have been predicted given the general theory and literature behind metal buffers.

### Example 8

### RT/PCR using HIV templates, Tricine and Bicine Buffers

A titration series of MnCl₂ concentration was used in RT/PCRs using HIV templates in both tricine and bicine buffers. Reactions were carried out in a 100 µl total reaction volume essentially as described in Example 10, below. Specific reaction conditions were as follows:
200 copies HIV cRNA (pNAS-2)
1 µg poly rA
13% glycerol (w/v)
150 µM each dATP, dCTP, dGTP, dTTP
200 µM dUTP
0.20 µM each SK431 (SEQ ID No. 6), SK462 (SEQ ID No. 5)
2 units UNG*
10 units rTth* DNA polymerase
65 mM KCl
50 mM Tricine-KOH (pH 8.3) or Bicine-KOH (pH 8.3)
MnCl₂ (1.0, 1.2, 1.5, 1.75, 2.0, 2.5 mM)

* Developed and manufactured by Hoffmann-La Roche Inc. and commercially available from Perkin Elmer, Norwalk, CT.

Reaction buffers contained 150 µM dTTP in addition to 200 µM dUTP because of the high percentage of adenine in the HIV RNA target and the reduced efficiency with which the Tth DNA polymerase incorporates dUMP during reverse transcription. The thermocycling profile was essentially as described in Example 10, except that the reverse transcription step was carried out at 70°C for 15 minutes. Amplified product was analyzed by gel electrophoresis as described in Example 10.

The target was found to be reverse transcribed and amplified in both the bicine and tricine buffers within a Mn²⁺ concentration range of from 1.0 to 2.5 mM, with higher levels of product formation found within a Mn²⁺ concentration range of from 1.2 to 2.0 mM.

### Example 9

### Increased dNTP Tolerance

To assess the increase in dNTP concentration tolerance using a bicine/KOAc/Mn(OAc)₂ buffer, RT/PCR amplifications of HCV cRNA target (pHCV1.1A) was carried out using both bicine and Tris buffers at varying dNTP concentrations. Reactions were carried out essentially as described in Example 10, below, except for modifications described herein.

HCV cRNA target is described in Example 6, above. Reactions were carried out in a 100 µl volume under the following conditions:
300 copies HCV cRNA
0.15 µM each KY78 (SEQ ID No. 16), KY90 (SEQ ID No. 17)
1 µg poly rA
8% glycerol
10 U rTth* DNA polymerase
2 U UNG*
dATP, dCTP, dGTP, and dUTP (100 µM each to 500 µM each)
50 mM Bicine-KOH (pH 8.3) or 10 mM Tris-HCl (pH 8.3)
100 mM KOAc (pH 7.5) or 90 mM KCl
2.5 mM Mn(OAc)₂ or 0.9 mM MnCl₂

* Developed and manufactured by Hoffmann-La Roche Inc. and commercially available from Perkin Elmer, Norwalk, CT.

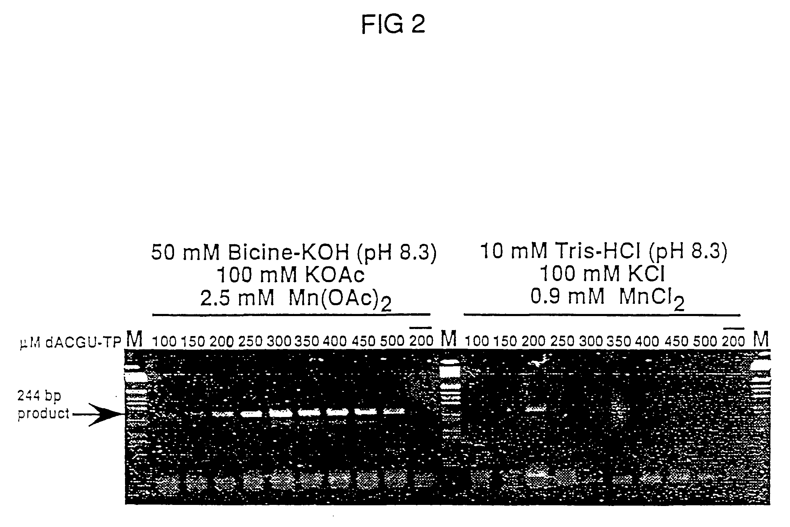

Thermocycling parameters were essentially as described in Example 10, below, except that the reverse transcription was at 70°C for 25 minutes and 40 amplification cycles were performed. Amplification product was analyzed by gel electrophoresis as described in Example 10. The results are shown in Figure 2.

The formation of amplification product using the bicine/KOAc/Mn(OAc)₂ buffer was observed over a range of dNTP concentration from 100 to 500 µM each dNTP. In contrast, significant levels of amplification product were formed using the Tris/KCl/MnCl₂ buffer only at a 200 µM each dNTP concentration.

### Example 10

### Homogeneous RT/PCR using HCV and HIV Templates

RT/PCR amplification based assays for the detection of hepatitis C virus (HCV) are described in European Patent Application EP-A-529,493, and in Young et al., 1993, supra.

EP-A-529,493 describes the detection of the amplified product using a microwell plate detection format. Similar assays are useful for the detection of human immunodeficiency virus (HIV). The homogeneous RT/PCR methods of the present invention are useful for the amplification of HIV and HCV viral templates using the protocols described below. Homogeneous reactions using both bicine/KOAc/Mn(OAc)₂ and Tris/KCl/MnCl₂ buffers are described below; the use of the bicine/KOAc/Mn(OAc)₂ buffer is preferred. Sample template may be either from clinical samples or the HIV and HCV templates described in Example 6, above. Suitable method for the preparation of clinical sample preparation are described in the above cited HCV assay references.

Preferred primers for the RT/PCR amplification of HIV templates are the 5' biotinylated derivatives of SK431 (SEQ ID No. 6) and SK462 (SEQ ID No. 5). Preferred primers for the RT/PCR amplification of HCV templates are KY78 (SEQ ID No. 16) and KY90 (SEQ ID No. 17).

Reaction conditions for HIV and HCV RT/PCR using Bicine-KOH (pH 8.3), KOAc (pH 7.5), and Mn(OAc)₂ in 100 µl total reaction volume are provided below. The HIV reaction conditions illustrate the use of an increased dUTP concentration to facilitate the incorporation of dUMP.

| For HIV templates: | For HCV templates: |
|---|---|
| 15% glycerol (w/v) | 10% glycerol (w/v) |
| 300 µM dATP | 200 µM dATP |
| 300 µM dCTP | 200 µM dCTP |
| 300 µM dGTP | 200 µM dGTP |
| 50 µM dTTP | |
| 500 µM dUTP | 200 µM dUTP |
| 20 pmol/rxn upstream primer | 15 pmol/rxn upstream primer |
| 20 pmol/rxn downstream primer | 15 pmol/rxn downstream primer |
| 2 units UNG* | 2 units UNG* |
| 10 units rTth* DNA polymerase | 15 units rTth* DNA polymerase |
| 50 mM Bicine-KOH pH 8.3 | 50 mM Bicine-KOH pH 8.3 |
| 100 mM KOAc pH 7.5 | 100 mM KOAc pH 7.5 |
| 3.6 mM Mn(OAc)₂ | 3.5 mM Mn(OAc)₂ |

| | |
|---|---|
| *Developed and manufactured by Hoffmann-La Roche Inc. and commercially available from Perkin Elmer, Norwalk, CT. | |

Reaction conditions for Tris-HCl (pH 8.3), KCl, MnCl₂ in 100 µl total reaction volume:

| For HIV templates: | For HCV templates: |
|---|---|
| 15% glycerol (w/v) | 10% glycerol (w/v) |
| 150 µM dATP | 200 µM dATP |
| 150 µM dCTP | 200 µM dCTP |
| 150 µM dGTP | 200 µM dGTP |
| 150 µM dTTP | |
| 200 µM dUTP | 200 µM dUTP |
| 20 pmol/rxn upstream primer | 15 pmol/rxn upstream primer |
| 20 pmol/rxn downstream primer | 15 pmol/rxn downstream primer |
| 2 units UNG∗ | 2 units UNG∗ |
| 10 units rTth∗ polymerase | 10 units rTth∗ polymerase |
| 90 mM KCl | 90 mM KCl |
| 10 mM Tris-HCl pH 8.3 | 10 mM Tris-HCl pH 8.3 |
| 0.85 mM MnCl₂ | 0.90 mM MnCl₂ |

| | |
|---|---|
| ∗ Developed and manufactured by Hoffmann-La Roche Inc. and commercially available from Perkin Elmer, Norwalk, CT. | |

Reactions are carried out in a TC9600 thermal cycler (Perkin Elmer, Norwalk, CT). The thermal cycler is programmed to provide the following temperature profile for the amplification of HIV template:
50°C for 2 minutes for the UNG sterilization;
60°C for 30 minutes for the reverse transcription step;
4 cycles of (95°C for 10 seconds, 55°C for 10 seconds, 72°C for 10 seconds);
24 cycles (Microwell Plate assay) or 36 cycles (Agarose Gel Analysis) of (90°C for 10 seconds, 60°C for 10 seconds, 72°C for 10 seconds); and
hold at 72°C.

The thermal cycler is programmed to provide the following temperature profile for the amplification of HCV template:
50°C for 2 minutes for the UNG sterilization;
60°C for 30 minutes for the reverse transcription;
2 cycles of 95°C for 15 seconds, 60°C for 20 seconds;
38 cycles of 90°C for 15 seconds, 60°C for 20 seconds;
60°C for 4 minutes; and
hold at 72°C.

Amplification product is analyzed either by visualization following agarose gel electrophoresis or by a microwell plate assay. For agarose gel analysis, 5 µl of each reaction are added to 2 µl of load buffer (30% sucrose, 0.1% bromophenol blue, 10 mM EDTA) and analyzed by 4% (3% NuSieve, 1% Agarose) agarose gel electrophoresis in 1X Tris-borate EDTA with ethidium bromide (10 µg per 100 ml of agarose) added into the agarose. Electrophoresis is at 125 V for 30 minutes.

Microwell plate analysis for HCV is described in EP-A-529,493 and in general, in U.S. Patent No. 5'232'829. Microwell plate analysis of HIV amplification product is as described for HCV but using the HIV specific probes described in Jackson et al, 1991, AIDS 5:1463-1467.

### Example 11

### RNA Stability

To assay the stability of RNA using different buffer conditions, RNA was incubated at elevated temperatures in reaction mixtures similar to those of a reverse transcription reaction but with the polymerase omitted to insure that no synthesis occurred. The reaction mixtures (20 µl volume each) consisted of the following:
100 ng [³³P] labeled pAW109 cRNA
1.5 µM KY80 (SEQ ID No. 17)
200 µM each dATP, dCTP, dGTP, and dUTP
2µl rTth DNA polymerase storage buffer (equivalent to 5 units of polymerase)

Buffer conditions were varied by the addition to the above reagents of the buffer reagents described below. All reaction mixtures were incubated at 70°C for 25 minutes with the exception of sample 1 which was incubate at 4°C for 25 minutes for comparison. The amount of full length RNA recovered was determined using an Ambis 4000 Radioanalytic Imaging System (Ambis, Inc., San Diego, CA) following gel electrophoresis. All values were normalized using the results of sample 2 as 100%. Bicine-KOH and Tris-HCI were added at pH 8.3; KOAc at pH 7.5.

| Added Reagents | Amount Recovered |
|---|---|
| 1. None added (4°C incubation) | 113% |
| 2. None added (70°C incubation) | 100% |
| 3. 2.5 mM Mn(OAc)₂ | 6% |
| 4. 2.5 mM Mn(OAc)₂; 100 mM KOAc | 1% |
| 5. 2.5 mM Mn(OAc)₂; 50 mM Bicine-KOH | 3% |
| 6. 2.5 mM Mn(OAc)₂; 100 mM KOAc; 50 mM Bicine-KOH | 25% |
| 7.∗ 2.0 mM Mn(OAc)₂; 100 mM KOAc; 50 mM Bicine KOH | 47% |
| 8. 2.5 mM Mn(OAc)₂; 100 mM KOAc; 50 mM Bicine KOH | 29% |
| 9. 3.0 mM Mn(OAc)₂; 100 mM KOAc; 50 mM Bicine KOH | 25% |
| 10. 0.9 mM MnCl₂ | 40% |
| 11. 1.0 mM MnCl₂ | 36% |
| 12. 1.0 mM MnCl₂; 90 mM KCl; 10 mM Tris-HCl | 16% |

| | |
|---|---|
| ∗ Sample 7 had an additional 100 µM of each dNTP (300 µM each dNTP). | |

The addition of manganese, which catalyzes the hydrolysis of RNA, increases the degradation of RNA at high temperatures as can be seen comparing samples 2 and 3. The addition of a buffer containing 2.5 mM Mn(OAc)₂, 100 mM KOAc, and 50 mM Bicine-KOH significantly reduced RNA degradation. Comparing samples 3, 4, 5, and 6 indicates that all components of the buffer must be present to decrease the amount of RNA degradation observed. The Mn(OAc)₂/KOAc/Bicine-KOH buffers decreased the amount of RNA degradation relative to the MnCl₂/KCl/Tris-HCl buffers as can be seen comparing samples 6-9 to sample 12.

A high temperature preincubation will facilitate the amplification of double-stranded RNA targets as well as targets with a high degree of secondary structure by denaturing the RNA prior to reverse transcription. To assess the effect of a high temperature preincubation on the stability of RNA in a bicine/KOAc/Mn(OAc)₂ buffer, RNA was incubated at elevated temperatures in reaction mixtures similar to those of a reverse transcription reaction but with the polymerase omitted to insure that no synthesis occurred. The reaction mixtures (20 µl volume each) consisted of the following:
250 ng [³³P] labeled pAW109 cRNA
1.5 µM DM151 (SEQ ID No. 10)
300 µM each dATP, dCTP, dGTP, and dUTP
50 mM Bicine-KOH (pH 8.3)
100 mM KOAc (pH 7.5)
2.5 mM Mn(OAc)₂
2 µl rTth DNA polymerase storage buffer (equivalent to 5 units of polymerase)

The reaction mixtures were incubated at the temperatures shown below and the final amount of full length RNA was determined using an Ambis 4000 Radioanalytic Imaging System (Ambis Inc., San Diego, CA) following gel electrophoresis. Incubations were done in triplicate and the average amount of undegraded RNA remaining was normalized to the amount remaining after a 25 minute 4°C incubation. The average standard deviation for the amount recovered from the 3 reaction incubations within a group was 11%.

| Incubation Temperature | Amount Recovered |
|---|---|
| 4°C for 25 minutes | 100% |
| 95°C for 15 seconds, 4°C for 25 minutes | 84% |
| 60°C for 25 minutes | 66% |
| 95°C for 15 seconds, 60°C for 25 minutes | 68% |

An incubation of 60°C for 25 minutes is comparable to the conditions of a reverse transcription as described in the previous examples. No detectable additional loss of full length labeled RNA occurred when a 15 second, 95°C preincubation of the RNA was included.

### Example 12

### Effect of Manganese Concentration on RT/PCR using HCV Templates

RT/PCR amplification reactions were carried out over a range of manganese concentrations using both bicine/KOAc/Mn(OAc)₂ and Tris/KCl/MnCl₂ buffers. Reaction conditions for the HCV RT/PCR in 100 µl total reaction volume are provided below.

| | |
|---|---|
| 100 copies HCV cRNA | |
| 200 µM dATP | |
| 200 µM dCTP | |
| 200 µM dGTP | |
| 200 µM dUTP | |
| 15 pmol/rxn KY78 (SEQ ID No. 16) | |
| 15 pmol/rxn KY90 (SEQ ID No. 17) | |
| 2 units UNG* | |
| 10 units rTth* DNA polymerase | |
| 8% glycerol (w/v) | |
| 50 mM Bicine-KOH (pH 8.3) | or 10 mM Tris-HCl (pH 8.3) |
| 100 mM KOAc (pH 7.5) | or 90 mM KCl |
| Mn(OAc)₂ | or MnCl₂ |

| | |
|---|---|
| * Developed and manufactured by Hoffmann-La Roche Inc. and commercially available from Perkin Elmer, Norwalk, CT. | |

The manganese concentrations used were 1.5, 2.0, 2.5, 3.0, 3.5, and 4.0 mM Mn(OAc)₂ and 0.7, 0.8, 0.85, 0.9, 0.95, and 1.0 mM MnCl₂. Reactions were carried out in a TC9600 thermal cycler (Perkin Elmer, Norwalk, CT). The thermal cycler was programmed to provide the temperature profile described in Example 10, with the exception that the reverse transcription was performed at 70°C for 25 minutes followed by a 1 minute incubation at 95°C. Amplification product was analyzed by visualization following agarose gel electrophoresis as described in Example 10.

Amplification product was observed using the bicine/KOAc/Mn(OAc)₂ buffer for a Mn(OAc)₂ concentration range of 2.0-4.0 mM. Amplification product was observed using the Tris/KCl/MnCl₂ buffer for a MnCl₂ concentration range of 0.8-1.0 mM. Under these reaction conditions, product was observed over a 10-fold greater range of manganese concentration using the bicine/KOAc/Mn(OAc)₂ buffer as compared to the Tris/KCl/MnCl₂ buffer.

### Example 13

### RT/PCR Using a High Tris Concentration

RT/PCR amplification reactions were carried out over a range of manganese concentrations using Tris/KCl/MnCl₂ buffers with two concentrations of Tris. Reaction conditions for the HCV RT/PCR in 100 µl total reaction volume are provided below.
500 copies HCV cRNA
200 µM dATP
200 µM dCTP
200 µM dGTP
200 µM dUTP
15 pmol/rxn KY78 (SEQ ID No. 16)
15 pmol/rxn KY80 (SEQ ID No. 17)
2 units UNG*
10 units rTth* DNA polymerase
8% glycerol (w/v)
10 mM Tris-HCl (pH 8.3) or 100 mM Tris-HCl (pH 8.3)
90 mM KCl or 45 mM KCl
MnCl₂

* Developed and manufactured by Hoffmann-La Roche Inc. and commercially available from Perkin Elmer, Norwalk, CT.

The manganese concentrations used were 0.7, 0.8, 1.0, 1.2 and 1.3 mM MnCl₂ for each Tris concentration. Reactions were carried out in a TC9600 thermal cycler (Perkin Elmer, Norwalk, CT). The thermal cycler was programmed to provide the temperature profile described in Example 10, with the exception that the reverse transcription was performed at 70°C for 25 minutes followed by a 1 minute incubation at 95°C. Amplification product was analyzed by visualization following agarose gel electrophoresis as described in Example 10.

Amplification product was observed using the 100 mM Tris buffer for a MnCl₂ concentration range of 0.7-1.2 mM. Amplification product was observed using the 10 mM Tris buffer for a MnCl₂ concentration range of 0.8-1.0 mM. Under these reaction conditions, product was observed over a greater range of manganese concentration when the concentration of Tris in a Tris/KCl/MnCl₂ buffer was increased from 10 to 100 mM.

The invention has been described in detail, but it will be understood that variations and modifications can be effected within the spirit and scope of the following claims.

## Claims

1. A method for amplifying a target RNA molecule in a sample, the method comprising the steps of:
(a) treating said sample in a reaction mixture comprising a first and second primer, wherein said first primer is sufficiently complementary to said target RNA to hybridize therewith and initiate synthesis of a cDNA molecule complementary to said target RNA, and said second primer is sufficiently homologous to said target RNA to hybridize to said cDNA and initiate synthesis of an extension product, and a thermostable DNA polymerase in the presence of all four deoxyribonucleoside triphosphates in a buffer, wherein said buffer comprises a divalent cation, at a temperature sufficient for said thermostable DNA polymerase to initiate synthesis of an extension product of said first primer to provide a cDNA molecule complementary to said target RNA;
(b) treating said reaction mixture at an appropriate temperature to provide single-stranded cDNA;
(c) treating said reaction mixture at an appropriate temperature for said thermostable DNA polymerase to initiate synthesis of an extension product of said second primer to provide a double-stranded cDNA molecule; and
(d) amplifying the double-stranded cDNA molecule of step (c) by a polymerase chain reaction;
**characterized in that** said buffers of steps (a) and (d) further comprise a buffering agent that binds said divalent cation, whereby said divalent cation is preferably Mn²⁺, wherein the K_{M} of the divalent cation binding reaction of said buffer at 20°C and 0.1 M ionic strength is between 10 and 10⁶, preferably between 10² and 10⁴, more preferably between 10^{2.5} and 10^{3.5}.

2. The method of Claim 1, wherein said buffering agent is a zwitterionic compound providing hydrogen ion buffering, wherein the pKₐ of said buffer at 20°C and 0.1 M ionic strength is between 7 and 9 , preferably between 7.5 and 8.5.

3. The method of Claim 1, wherein said buffer comprises N,N-bis(2-hydroxyethyl)glycine or N[tris(hydroxymethyl)methyl]glycine.

4. The method of Claim 3, wherein said buffer further comprises an acetate salt selected from the group consisting of sodium acetate, potassium acetate, ammonium acetate, and lithium acetate.

5. The method of any one of Claims 1 to 4, wherein said thermostable DNA polymerase is the Thermus aquaticus DNA polymerase or the Thermus thermophilus DNA polymerase.

6. The method of Claim 5, wherein said DNA polymerase is recombinant Tth DNA polymerase.

7. The method of any one of Claims 1 to 6, wherein the temperature at step (a) is between 40°C and 80°C.

8. A method of sterilizing a reverse transcription reaction, a homogeneous reverse transcription/amplification reaction or an amplification reaction contaminated with nucleic acids generated from a previous reverse transcription reaction, wherein said previous reverse transcription reaction resulted from mixing conventional and unconventional nucleoside triphosphates into a reverse transcription reaction mixture and generating cDNA products having the conventional and unconventional nucleotides incorporated therein, which method comprises degrading the contaminating nucleic acids by hydrolyzing covalent bonds of the unconventional nucleotides and wherein said reverse transcription reaction mixture further comprises a Thermus thermophilus DNA polymerase, a divalent cation, preferably Mn²⁺, and a buffer, wherein said buffer comprises a buffering agent that binds said divalent cation, wherein the K_{M} of the divalent cation binding reaction of said buffer at 20°C and 0.1 M ionic strength is between 10 and 10⁶, preferably between 10² and 10⁴, more preferably between 10^{2.5} and 10^{3.5}.

9. The method of Claim 8, wherein said previous reverse transcription reaction is a homogeneous reverse transcription/amplification reaction.

10. The method of Claim 8 or Claim 9, wherein said buffering agent is a zwitterionic compound providing hydrogen ion buffering, wherein the pKₐ of said buffer at 20°C and 0.1 M ionic strength is between 7 and 9, preferably between 7.5 and 8.5.

11. The method of Claim 8 or Claim 9, wherein said buffer comprises N,N-bis(2-hydroxyethyl)glycine or N[tris(hydroxymethyl)methyl]glycine.

12. The method of Claim 11, wherein said buffer further comprises an acetate salt selected from the group consisting of sodium acetate, potassium acetate, ammonium acetate, and lithium acetate.

13. A buffer for carrying out a homogeneous reverse transcription/amplification reaction comprising a divalent cation, a monovalent cation, and a buffering agent, wherein the divalent cation is preferably Mn²⁺ and the buffering agent is a chelating agent that binds said divalent cation, wherein the K_{M} of the divalent cation binding reaction of said buffering agent at 20°C and 0.1 M ionic strength is between 10 and 10⁶, preferably between 10² and 10⁴, more preferably between 10^{2.5} and 10^{3.5}.

14. The buffer of Claim 13, wherein said buffering agent is a zwitterionic compound providing hydrogen ion buffering, wherein the pKₐ of said buffer at 20°C and 0.1 M ionic strength is between 7 and 9, preferably between 7.5 and 8.5.

15. The buffer of Claim 13, wherein the buffering agent is N,N-bis(2-hydroxyethyl)glycine or N[tris(hydroxymethyl)methyl]glycine.

16. The buffer of Claim 15, wherein said divalent cation is supplied by manganese acetate, manganese chloride or manganese sulfate, and the monovalent cation is supplied by an acetate salt selected from the group consisting of sodium acetate, potassium acetate, ammonium acetate, and lithium acetate.

17. The buffer of Claim 16, wherein said divalent cation is supplied by manganese acetate, at a concentration of between 1.2 and 5 mM.

18. The buffer of Claim 16, wherein the monovalent cation is supplied by potassium acetate.

19. A method for reverse transcribing a target RNA molecule in a sample, the method comprising the steps of: treating said sample in a reaction mixture comprising a primer, wherein said primer is sufficiently complementary to said target RNA to hybridize therewith and initiate synthesis of a cDNA molecule complementary to said target RNA, a thermoactive DNA polymerase, four deoxyribonucleoside triphosphates, and an appropriate buffer, wherein said buffer comprises a divalent cation, preferably Mn²⁺, at a temperature sufficient for said thermoactive DNA polymerase to initiate synthesis of an extension product of said primer to provide a cDNA molecule complementary to said target RNA, wherein said buffer further comprises a buffering agent that binds said divalent cation, wherein the KM of the divalent cation binding reaction of said buffer at 20°C and 0.1 M ionic strength is between 10 and 10⁶, preferably between 10² and 10⁴, more preferably between 10^{2.5} and 10^{3.5}.

20. The method of Claim 19, wherein said buffering agent is a zwitterionic compound providing hydrogen ion buffering, wherein the pKₐ of said buffer at 20°C and 0.1 M ionic strength is between 7 and 9, preferably between 7.5 and 8.5.

21. The method of Claim 20, wherein said buffer comprises N,N-bis(2-hydroxyethyl)glycine or N[tris(hydroxymethyl)methyl]glycine.

22. The method of Claim 20, wherein said buffer comprises N,N-bis(2-hydroxyethyl)glycine or N[tris(hydroxymethyl)methyl]glycine; and wherein said buffer further comprises an acetate salt selected from the group consisting of sodium acetate, potassium acetate, ammonium acetate, and lithium acetate.

23. The method of Claim 22, wherein said thermoactive DNA polymerase is the Thermus aquaticus DNA polymerase or the Thermus thermophilus DNA polymerase.

24. The method of Claim 23, wherein said DNA polymerase is recombinant Tth DNA polymerase.

25. The method of Claim 23 or Claim 24, wherein said temperature of said reaction mixture is between 40°C and 80°C.

26. Use of a buffer as claimed in Claims 13 to 18 for performing a reverse transcription reaction using a thermostable DNA polymerase.

27. A kit comprising a buffer as claimed in Claims 13 to 18 and a thermoactive DNA polymerase.

## Patentansprüche

1. Verfahren zur Amplifikation eines Ziel-RNA-Moleküls in einer Probe, wobei das Verfahren die folgenden Schritte umfaßt:
a) Behandeln der Probe in einem Reaktionsgemisch, umfassend einen ersten und zweiten Primer, wobei der erste Primer ausreichend komplementär zu der Ziel-RNA ist, um damit zu hybridisieren und die Synthese eines cDNA-Moleküls zu veranlassen, das komplementär zu der Ziel-RNA ist, und der zweite Primer ausreichend homolog zu der Ziel-RNA ist, um mit der cDNA zu hybridisieren und die Synthese eines Verlängerungsprodukts zu veranlassen, und eine hitzestabile DNA-Polymerase in Gegenwart aller 4 Desoxyribonucleosidtriphosphate in einem Puffer, wobei der Puffer ein divalentes Kation umfaßt, bei einer Temperatur, die ausreicht, damit die hitzestabile DNA-Polymerase die Synthese eines Verlängerungsprodukts des ersten Primers veranlassen kann, um ein cDNA-Molekül bereitzustellen, das komplementär zu der Ziel-RNA ist;
b) Behandeln des Reaktionsgemisches bei einer geeigneten Temperatur, um einzelsträngige cDNA bereitzustellen;
c) Behandeln des Reaktionsgemisches bei einer geeigneten Temperatur, damit die hitzestabile DNA-Polymerase die Synthese eines Verlängerungsproduktes des zweiten Primers veranlassen kann, um ein doppelsträngiges cDNA-Molekül bereitzustellen; und
d) Amplifikation des doppelsträngigen cDNA-Moleküls von Schritt c) durch eine Polymerasekettenreaktion;
**dadurch gekennzeichnet, daß** die Puffer der Schritte a) und d) ferner ein Pufferungsmittel umfassen, das das divalente Kation bindet, wobei das divalente Kation vorzugsweise Mn²⁺ ist, wobei der K_{M}-Wert der Bindungsreaktion des divalenten Kations des Puffers bei 20° C und 0,1 M lonenstärke zwischen 10 und 10⁶, vorzugsweise zwischen 10² und 10⁴, mehr bevorzugt zwischen 10^{2,5} und 10^{3,5} liegt.

2. Verfahren nach Anspruch 1, wobei das Pufferungsmittel eine zwitterionische Verbindung ist, die Wasserstoffionenpufferung bereitstellt, wobei der pKₐ-Wert des Puffers bei 20° C und 0,1 M lonenstärke zwischen 7 und 9, vorzugsweise zwischen 7,5 und 8,5 liegt.

3. Verfahren nach Anspruch 1, wobei der Puffer N,N-Bis(2-hydroxyethyl)glycin oder N[Tris(hydroxymethyl)methyl]glycin umfaßt.

4. Verfahren nach Anspruch 3, wobei der Puffer ferner ein Acetatsalz umfaßt, ausgewählt aus der Gruppe bestehend aus Natriumacetat, Kaliumacetat, Ammoniumacetat und Lithiumacetat.

5. Verfahren nach einem der Ansprüche 1 bis 4, wobei die hitzestabile DNA-Polymerase die Thermus aquaticus-DNA-Polymerase oder die Thermus thermophilus-DNA-Polymerase ist.

6. Verfahren nach Anspruch 5, wobei die DNA-Polymerase die rekombinante Tth-DNA-Polymerase ist.

7. Verfahren nach einem der Ansprüche 1 bis 6, wobei die Temperatur in Schritt a) zwischen 40° C und 80° C liegt.

8. Verfahren zum Sterilisieren einer reversen Transkriptionsreaktion, einer homogenen reversen Transkription/Amplifikationsreaktion oder einer Amplifikationsreaktion, die mit Nucleinsäuren verunreinigt sind, die von einer vorangehenden reversen Transkriptionsreaktion stammen, wobei die vorangehende Transkriptionsreaktion vom Mischen üblicher und unüblicher Nucleosidtriphosphate in ein reverses Transkripitionsreaktionsgemisch und der Erzeugung von cDNA-Produkten mit darin eingebauten üblichen und unüblichen Nucleotiden herrührt, wobei das Verfahren den Abbau der verunreinigenden Nucleinsäuren durch Hydrolyse kovalenter Bindungen der unüblichen Nucleotide umfaßt und wobei das reverse Transkriptionsreaktionsgemisch ferner eine Thermus thermophilus-DNA-Polymerase, ein divalentes Kation, vorzugsweise Mn²⁺, und einen Puffer umfaßt, wobei der Puffer ein Pufferungsmittel umfaßt, das das divalente Kation bindet, wobei der KM-Wert der Bindungsreaktion des divalenten Kations des Puffers bei 20° C und 0,1 M lonenstärke zwischen 10 und 10⁶, vorzugsweise zwischen 10² und 10⁴, mehr bevorzugt zwischen 10^{2,5} und 10^{3,5} liegt.

9. Verfahren nach Anspruch 8, wobei die vorangehende reverse Transkriptionsreaktion eine homogene reverse Transkriptions/Amplifikationsreaktion ist.

10. Verfahren nach Anspruch 8 oder 9, wobei das Pufferungsmittel eine zwitterionische Verbindung ist, die Wasserstoffionenpufferung bereitstellt, wobei der pKₐ-Wert des Puffers bei 20° C und 0,1 M Ionenstärke zwischen 7 und 9, vorzugsweise zwischen 7,5 und 8,5 liegt.

11. Verfahren nach Anspruch 8 oder 9, wobei der Puffer N,N-Bis(2-hydroxyethyl)glycin oder N[Tris(hydroxymethyl)methyl]glycin umfaßt.

12. Verfahren nach Anspruch 11, wobei der Puffer ferner ein Acetatsalz umfaßt, ausgewählt aus der Gruppe bestehend aus Natriumacetat, Kaliumacetat, Ammoniumacetat und Lithiumacetat.

13. Puffer zur Durchführung einer homogenen reversen Transkriptions/Amplifikationsreaktion, umfassend ein divalentes Kation, ein monovalentes Kation und ein Pufferungsmittel, wobei das divalente Kation vorzugsweise Mn²⁺ ist und das Pufferungsmittel ein Chelatbildner ist, der das divalente Kation bindet, wobei der KM-Wert der Bindungsreaktion des divalenten Kations des Pufferungsmittels bei 20° C und 0,1 M lonenstärke zwischen 10 und 10⁶, vorzugsweise zwischen 10² und 10⁴, mehr bevorzugt zwischen 10^{2,5} und 10^{3,5} liegt.

14. Puffer nach Anspruch 13, wobei das Pufferungsmittel eine zwitterionische Verbindung ist, die Wasserstoffionenpufferung bereitstellt, wobei der pKₐ-Wert des Puffers bei 20° C und 0,1 M lonenstärke zwischen 7 und 9, vorzugsweise zwischen 7,5 und 8,5 liegt.

15. Puffer nach Anspruch 13, wobei das Pufferungsmittel N,N-Bis(2-hydroxyethyl)glycin oder N[Tris(hydroxymethyl)methyl]glycin ist.

16. Puffer nach Anspruch 15, wobei das divalente Kation durch Manganacetat, Manganchlorid oder Mangansulfat und das monovalente Kation durch ein Acetatsalz bereitgestellt wird, ausgewählt aus der Gruppe bestehend aus Natriumacetat, Kaliumacetat, Ammoniumacetat und Lithiumacetat.

17. Puffer nach Anspruch 16, wobei das divalente Kation durch Manganacetat bei einer Konzentration zwischen 1,2 und 5 mM bereitgestellt wird.

18. Puffer nach Anspruch 16, wobei das monovalente Kation durch Kaliumacetat bereitgestellt wird.

19. Verfahren zur reversen Transkription eines Ziel-RNA-Moleküls in einer Probe, wobei das Verfahren die folgenden Schritte umfaßt:
Behandeln der Probe in einem Reaktionsgemisch, das einen Primer umfaßt, wobei der Primer ausreichend komplementär zu der Ziel-RNA ist, um damit zu hybridisieren und die Synthese eines cDNA-Moleküls zu veranlassen, das komplementär zu der Ziel-RNA ist, eine hitzeaktive DNA-Polymerase, 4 Desoxyribonucleosidtriphosphate und einen geeigneten Puffer, wobei der Puffer ein divalentes Kation umfaßt, vorzugsweise Mn²⁺, bei einer Temperatur, die ausreicht, damit die hitzeaktive DNA-Polymerase die Synthese eines Verlängerungsprodukts des Primers veranlassen kann, um ein cDNA-Molekül bereitzustellen, das komplementär zu der Ziel-RNA ist, wobei der Puffer ferner ein Pufferungsmittel umfaßt, das das divalente Kation bindet, wobei der K_{M}-Wert der Bindungsreaktion des divalenten Kations des Puffers bei 20° C und 0,1 M lonenstärke zwischen 10 und 10⁶, vorzugsweise zwischen 10² und 10⁴, mehr bevorzugt zwischen 10^{2,5} und 10^{3,5} liegt.

20. Verfahren nach Anspruch 19, wobei das Pufferungsmittel eine zwitterionische Verbindung ist, die Wasserstoffionenpufferung bereitstellt, wobei der pKₐ-Wert bei 20° C und 0,1 M lonenstärke zwischen 7 und 9, vorzugsweise zwischen 7,5 und 8,5 liegt.

21. Verfahren nach Anspruch 20, wobei der Puffer N,N-Bis(2-hydroxyethyl)glycin oder N[Tris(hydroxymethyl)methyl]glycin umfaßt.

22. Verfahren nach Anspruch 20, wobei der Puffer N,N-Bis(2-hydroxyethyl)glycin oder N[Tris(hydroxymethyl)methyl]glycin umfaßt und wobei der Puffer ferner ein Acetatsalz umfaßt, ausgewählt aus der Gruppe bestehend aus Natriumacetat, Kaliumacetat, Ammoniumacetat und Lithiumacetat.

23. Verfahren nach Anspruch 22, wobei die hitzeaktive DNA-Polymerase die Thermus aquaticus-DNA-Polymerase oder die Thermus thermophilus-DNA-Polymerase ist.

24. Verfahren nach Anspruch 23, wobei die DNA-Polymerase die rekombinante Tth-DNA-Polymerase ist.

25. Verfahren nach Anspruch 23 oder 24, wobei die Temperatur des Reaktionsgemisches zwischen 40° C und 80° C liegt.

26. Verwendung eines Puffers nach Anspruch 13 bis 18 für die Durchführung einer reversen Transkriptionsreaktion unter Verwendung einer hitzestabilen DNA-Polymerase.

27. Kit, umfassend einen Puffer nach Anspruch 13 bis 18 und eine hitzeaktive DNA-Polymerase.

## Revendications

1. Procédé pour amplifier une molécule d'ARN cible dans un échantillon, le procédé comprenant les étapes :
(a) de traitement dudit échantillon dans un milieu réactionnel comprenant une première et une deuxième amorces, où ladite première amorce est suffisamment complémentaire dudit ARN cible pour s'hybrider avec ce dernier et déclencher la synthèse d'une molécule d'ADNc complémentaire dudit ARN cible, et ladite deuxième amorce est suffisamment homologue dudit ARN cible pour s'hybrider audit ADNc et déclencher la synthèse d'un produit d'allongement, et une ADN-polymérase thermostable, en présence des quatre désoxyribonucléosidetriphosphates dans un tampon, où ledit tampon comprend un cation divalent, à une température suffisante pour que ladite ADN-polymérase thermostable déclenche la synthèse d'un produit d'allongement de ladite première amorce, pour fournir une molécule d'ADNc complémentaire dudit ARN cible ;
(b) de traitement dudit mélange réactionnel à une température appropriée, pour fournir un ADNc simple brin ;
(c) de traitement dudit mélange réactionnel, à une température appropriée, pour que ladite ADN-polymérase thermostable déclenche la synthèse d'un produit d'allongement de ladite deuxième amorce, pour fournir une molécule d'ADNc double brin ; et
(d) d'amplification de la molécule d'ADNc double brin de l'étape (c) par une réaction en chaîne par polymérase ;
**caractérisé en ce que** lesdits tampons des étapes (a) et (d) comprennent en outre un agent de tamponnage, qui se lie audit cation divalent, ledit cation divalent étant de préférence Mn²⁺, où le K_{M} de la réaction de liaison du cation divalent dudit tampon à 20°C et pour une force ionique de 0,1 M est compris entre 10 et 10⁶, de préférence entre 10² et 10⁴, plus particulièrement entre 10^{2,5} et 10^{3,5}.

2. Procédé selon la revendication 1, dans lequel ledit agent de tamponnage est un composé zwittérionique assurant le tamponnage de l'ion hydrogène, où le pKₐ dudit tampon à 20°C pour une force ionique de 0,1 M est compris entre 7 et 9 et de préférence entre 7,5 et 8,5.

3. Procédé selon la revendication 1, dans lequel ledit tampon contient de la N,N-bis(2-hydroxyéthyl)glycine ou de la N-[tris(hydroxyméthyl)méthyl]glycine.

4. Procédé selon la revendication 3, dans lequel ledit tampon comprend en outre un sel acétate choisi dans l'ensemble comprenant l'acétate de sodium, l'acétate de potassium, l'acétate d'ammonium et l'acétate de lithium.

5. Procédé selon l'une quelconque des revendications 1 à 4, dans lequel ladite ADN-polymérase thermostable est l'ADN-polymérase de Thermus aquaticus ou l'ADN-polymérase de Thermus thermophilus.

6. Procédé selon la revendication 5, dans lequel ladite ADN-polymérase est une ADN-polymérase Tth recombinante.

7. Procédé selon l'une quelconque des revendications 1 à 6, dans lequel la température dans l'étape (a) est comprise entre 40 et 80°C.

8. Procédé de stérilisation d'une masse réactionnelle de réaction de transcription inverse, d'une masse réactionnelle de réaction de transcription inverse homogène/ amplification ou d'une masse réactionnelle de réaction d'amplification contaminée par des acides nucléiques produits lors d'une réaction antérieure de transcription inverse, dans lequel ladite réaction antérieure de transcription inverse résulte du mélange de nucléosidetriphosphates classiques et non-classiques, qui donne un mélange réactionnel de réaction de transcription inverse, et de la production de produits d'ADNc dans lesquels sont incorporés les nucléotides classiques et non-classiques, lequel procédé comprend la dégradation des acides nucléiques contaminants par hydrolyse des liaisons covalentes des nucléotides non-classiques, et où ledit mélange réactionnel de réaction de transcription inverse comprend en outre une ADN-polymérase de Thermus thermophilus, un cation divalent, de préférence Mn²⁺, et un tampon, où le K_{M} de la réaction de liaison du cation divalent dudit tampon à 20°C et pour une force ionique de 0,1 M est compris entre 10 et 10⁶, de préférence entre 10² et 10⁴, plus particulièrement entre 10^{2,5} et 10^{3,5}.

9. Procédé selon la revendication 8, dans lequel ladite réaction antérieure de transcription inverse est une réaction de transcription inverse homogène/amplification.

10. Procédé selon la revendication 8 ou 9, dans lequel ledit agent de tamponnage est un composé zwittérionique assurant le tamponnage de l'ion hydrogène, où le pKₐ dudit tampon à 20°C pour une force ionique de 0,1 M est compris entre 7 et 9 et de préférence entre 7,5 et 8,5.

11. Procédé selon la revendication 8 ou 9, dans lequel ledit tampon contient de la N,N-bis(2-hydroxyéthyl)-glycine ou de la N-[tris(hydroxyméthyl)méthyl]glycine.

12. Procédé selon la revendication 11, dans lequel ledit tampon comprend en outre un sel acétate choisi dans l'ensemble comprenant l'acétate de sodium, l'acétate de potassium, l'acétate d'ammonium et l'acétate de lithium.

13. Tampon pour mettre en oeuvre une réaction de transcription inverse homogène/amplification, comprenant un cation divalent, un cation monovalent et un agent de tamponnage, où le cation divalent est de préférence Mn²⁺, où le K_{M} de la réaction de liaison du cation divalent dudit tampon à 20°C et pour une force ionique de 0,1 M est compris entre 10 et 10⁶, de préférence entre 10² et 10⁴, plus particulièrement entre 10^{2,5} et 10^{3,5}.

14. Tampon selon la revendication 13, dans lequel ledit agent de tamponnage est un composé zwittérionique assurant le tamponnage de l'ion hydrogène, où le pKₐ dudit tampon à 20°C pour une force ionique de 0,1 M est compris entre 7 et 9 et de préférence entre 7,5 et 8,5.

15. Tampon selon la revendication 13, dans lequel ledit tampon contient de la N,N-bis(2-hydroxyéthyl)glycine ou de la N-[tris(hydroxyméthyl)méthyl]glycine.

16. Tampon selon la revendication 15, dans lequel ledit cation divalent est fourni par l'acétate de manganèse, le chlorure de manganèse ou le sulfate de manganèse, et le cation monovalent est fourni par un sel acétate choisi dans l'ensemble comprenant l'acétate de sodium, l'acétate de potassium, l'acétate d'ammonium et l'acétate de lithium.

17. Tampon selon la revendication 16, dans lequel ledit cation divalent est fourni par l'acétate de magnésium à une concentration comprise entre 1,2 et 5 mM.

18. Tampon selon la revendication 16, dans lequel le cation monovalent est fourni par l'acétate de potassium.

19. Procédé pour la transcription inverse d'une molécule d'ARN cible dans un échantillon, comprenant les étapes de traitement dudit échantillon dans un mélange réactionnel comprenant une amorce, où ladite amorce est suffisamment complémentaire dudit ARN cible pour s'hybrider avec ce dernier et déclenche la synthèse d'une molécule d'ADNc complémentaire dudit ARN cible, une ADN-polymérase thermoactive, quatre désoxyribonucléosidetriphosphates et un tampon approprié, où ledit tampon comprend un cation divalent, de préférence Mn²⁺, à une température suffisante pour que ladite ADN-polymérase thermoactive déclenche la synthèse d'un produit d'allongement de ladite amorce pour fournir une molécule d'ADNc complémentaire dudit ARN cible, où ledit tampon comprend en outre un agent de tamponnage qui se lie audit cation divalent, où le K_{M} de la réaction de liaison du cation divalent dudit tampon à 20°C et pour une force ionique de 0,1 M est compris entre 10 et 10⁶, de préférence entre 10² et 10⁴, plus particulièrement entre 10^{2,5} et 10^{3,5}.

20. Procédé selon la revendication 19, dans lequel ledit agent de tamponnage est un composé zwittérionique assurant le tamponnage de l'ion hydrogène, où le pKₐ dudit tampon à 20°C pour une force ionique de 0,1 M est compris entre 7 et 9 et de préférence entre 7,5 et 8,5.

21. Procédé selon la revendication 20, dans lequel ledit tampon contient de la N,N-bis(2-hydroxyéthyl)glycine ou de la N-[tris(hydroxyméthyl)méthyl]glycine.

22. Procédé selon la revendication 20, dans lequel ledit tampon contient de la N,N-bis(2-hydroxyéthyl)glycine ou de la N-[tris(hydroxyméthyl)méthyl]glycine ; et dans lequel ledit tampon comprend en outre un sel acétate choisi dans l'ensemble comprenant l'acétate de sodium, l'acétate de potassium, l'acétate d'ammonium et l'acétate de lithium.

23. Procédé selon la revendication 22, dans lequel ladite ADN-polymérase thermoactive est l'ADN-polymérase de Thermus aquaticus ou l'ADN-polymérase de Thermus thermophilus.

24. Procédé selon la revendication 23, dans lequel ladite ADN-polymérase est une ADN-polymérase de Tth recombinante.

25. Procédé selon la revendication 23 ou 24, dans lequel ladite température dudit mélange réactionnel est comprise entre 40 et 80°C.

26. Utilisation d'un tampon selon les revendications 13 à 18 pour mettre en oeuvre une réaction de transcription inverse par utilisation d'une ADN-polymérase thermostable.

27. Trousse comprenant un tampon selon les revendications 13 à 18 et une ADN-polymérase thermoactive.
